# EUROPEAN PATENT APPLICATION

(11) **EP 3 572 525 A2**
(43) Date of publication of application: **27.11.2019**
(21) Application number: 18741209.3
(22) Date of filing: 16.01.2018
(51) Int. Cl.: C12Q 1/68, C12N 9/22, C12N 9/78

(54) **METHOD FOR IDENTIFYING BASE EDITING OFF-TARGET SITE BY DNA SINGLE STRAND BREAK**

(30) Priority: 17.01.2017 US 201762446951 P
(71) Applicant: Institute for Basic Science, Daejeon 34047 (KR)
(72) Inventor: KIM, Jin-Soo, Seoul 08831 (KR)
(74) Representative: Bates, Philip Ian
(86) International application number: PCT/KR2018/000747
(87) International publication number: WO 2018/135838

(57) **Abstract**

Provided are a DNA single strand break composition comprising cytidine deaminase, inactivated target-specific endonuclease, and guide RNA, a method for producing a DNA single strand break, using the same, a DNA nucleotide sequencing method with base editing introduced thereto, and a method for identifying (or measuring or detecting) a base editing position, base editing efficiency on an on-target site and an off-target site, and/or target specificity.

## Description

### BACKGROUND OF THE INVENTION

### 1. Field

The present disclosure relates to a composition for inducing DNA single strand breaks in DNA, the composition comprising a cytidine deaminase, an inactivated target-specific endonuclease, and a guide RNA; a method for inducing a single-strand break in DNA, using the same; a method for analyzing a nucleic acid sequence of a base-editing-introduced DNA; and a method for identifying (or measuring or detecting) a base-editing site, base-editing efficiency at an on-target site, an off-target site, and/or target specificity.

### 2. Description of the Related Art

A base editor (programmable deaminase) comprising a DNA-binding module and a cytidine deaminase enables targeted nucleotide substitutions or base editing in a genome without producing DNA-strand breaks. Unlike programmable nucleases, such as CRISPR-Cas9 and ZFN (zinc-finger nuclease), which induce small insertions or deletions (indels) at a target site, programmable deaminases convert C to T (C to G or A, to a lesser extent) within several nucleotides at a target site. Programmable deaminases can correct point mutations causing genetic diseases or can create single-nucleotide polymorphisms (SNPs) of interest in human cells, animals, and plants.

To data, four different classes of programmable deaminases have been reported: 1) Base Editors (BEs) composed of catalytically-deficient Cas9 (dCas9) derived from *S. pyogenes* or D10A nickase (nCas9) and rAPOBEC1, a cytidine deaminase from rats; 2) Target-AID composed of dCas9 or nCas9 and PmCDA1, an activation-induced cytidine deaminase (AID) ortholog from sea lamprey2, or human AID; 3) CRISPR-X composed of dCas9 and sgRNAs linked to MS2 RNA hairpins to recruit a hyperactive AID variant fused to MS2-binding protein; and 4) Zinc-finger proteins or transcription activator-like effectors (TALEs) fused to a cytidine deaminase.

In spite of broad interest in base editing with a base editor, appropriate methods have not yet been developed for analyzing genome-wide target specificities of programmable deaminases. There is therefore a need for the development of a tool capable of analyzing genome-wide target specificities of base editors, thereby analyzing the base editors for base editing efficiency, off-target sites, and off-target effects.

### SUMMARY OF THE INVENTION

Provided in the present specification are a means for analyzing genome-wide target specificities of a base editor and a means for analyzing off-target sites and off-target effects of the base editor through the analysis of genome-wide target specificities.

An aspect provides a composition for producing single-strand breaks in DNA, the composition comprising: (a) a deaminase or a gene coding therefor (cDNA, rDNA, or mRNA); (b) an inactivated target-specific endonuclease or a gene coding therefor (cDNA, rDNA, or mRNA); and (c) a guide RNA or a gene coding therefor. The composition may not contain a uracil-specific excision reagent (USER).

Another aspect provides a method for inducing a single-strand break in DNA, the method comprising a step of introducing into a cell or contacting with DNA separated from cells, (a) a deaminase or a gene coding therefor (cDNA, rDNA, or mRNA), (b) an inactivated target-specific endonuclease or a gene coding therefor (cDNA, rDNA, or mRNA), and (c) a guide RNA or a gene coding therefor. This method may not comprise a step of treating with a Uracil-Specific Excision Reagent (USER).

Another aspect provides a method for analyzing a nucleic acid sequence of DNA to which base editing is introduced by deaminase, the method comprising the steps of:
(i) introducing into a cell or contacting with DNA separated from cells (a) a deaminase or a gene coding therefor (cDNA, rDNA, or mRNA), (b) an inactivated target-specific endonuclease or a gene coding therefor(cDNA, rDNA, or mRNA), and (c) a guide RNA or a gene coding therefor to induce a single-strand break in the DNA; and
(ii) analyzing a nucleic acid sequence of a DNA fragment in which the single-strand break has been induced. The method may not comprise a step of treating with a uracil-specific excision reagent (USER) to produce a double-strand break in DNA.

Another aspect provides a method for identifying (or measuring or detecting) a base-editing site, a single-strand break site, base editing efficiency at an on-target site, an off-target site, and target specificity of deaminase, the method comprising the steps of:
(i) introducing to a cell or contacting with DNA separated from cells (a) a deaminase or a gene coding therefor (cDNA, rDNA, or mRNA), (b) an inactivated target-specific endonuclease or a gene coding therefor(cDNA, rDNA, or mRNA), and (c) a guide RNA or a gene coding therefor to induce a single-strand break in DNA;
(ii) analyzing a nucleic acid sequence of the cleaved DNA fragment; and
(iii) identifying a single-strand break site from the nucleic acid sequence data obtained by the analysis. The method may further comprise, for example, the step of (iii-1) identifying base editing (e.g., conversion of cytosine (C) to uracil (U) or thymine (T)) in the nucleic acid sequence data obtained by the analysis (sequence read) between steps (ii) and (iii) or concomitantly with, prior to or after step (iii). The method may not comprise a step of treating with a uracil-specific excision reagent (USER) to induce a double-strand break in DNA. In one embodiment, the method (for identifying, for example, base editing efficiency at an on-target site, and an off-target site) may further comprise, after step (iii), a step of (iv) identifying (determining) the break site as an off-target site when the break site is not within an on-target site.

### DETAILED DESCRIPTION

In the specification, Digenome-seq is modified to assess the specificity of a base sequence (e.g., Base Editor 3; BE3) composed of a Cas9 nickase and a deaminase in the human genome. Genomic DNA is treated with BE3 and a guide RNA *in vitro* to identify the production of a break in a single strand of the DNA double helix. BE3 off-target sites are then computationally identified from whole genome sequencing data by a method for inducing a single-strand break in DNA, using a deaminase and a method for analyzing a nucleic acid sequence, both provided in the present specification.

First of all, provided is a technique of producing double-strand breaks in DNA by using a deaminase which does not induce a double-strand break.

Another aspect provides a composition for inducing a single-strand break in DNA, the composition comprising (a) a deaminaseor a gene coding therefor (cDNA, rDNA, or mRNA), (b) an inactivated target-specific endonuclease or a gene coding therefor(cDNA, rDNA, or mRNA), and (c) a guide RNA or a gene coding therefor. The composition may not contain a uracil-specific excision reagent (USER).

The encoding gene used in the present specification may be used in the form of cDNA, rDNA or a recombinant vector carrying the same, or mRNA.

The deaminase may be cytidine deaminase. The term "cytidine deaminase", as used herein, is intended to encompass all enzymes that have the activity of converting cytosine, which is a base existing in nucleotides (e.g., double-strand DNA, or RNA) to uracil (C-to-U conversion or C-to-U editing). The cytidine that the cytidine deaminase converts to uracil is present on a strand having PAM sequence in the sequence at an on-target site (on-target sequence). In an embodiment, the cytidine deaminase may be derived from mammals including primates such as humans, apes, etc., and rodents such as rats, mice, etc., but is not limited thereto. For example, the cytidine deaminase may be at least one selected from the group consisting of members of an APOBEC ("apolipoprotein B mRNA editing enzyme, catalytic polypeptide-like") family, AID (activation-induced cytidine deaminase), and CDA (cytidine deaminase; e.g., CDA1), and specifically from, but not limited to, the following group:
APOBEC1: *Homo sapiens* APOBEC1 (proteins: GenBank Accession Nos. NP_001291495.1, NP_001635.2, and NP_005880.2; genes (as used herein, genes may refer to mRNA or cDNA) (genes encoding the proteins previously described are filled in the same order as in the proteins): GenBank Accession Nos. NM_001304566.1, NM_001644.4, and NM_005889.3), mouse (*Mus musculus*) APOBEC1 (proteins: GenBank Accession Nos. NP_001127863.1, and NP_112436.1; genes (genes encoding the proteins previously described are filled in the same order as in the proteins): GenBank Accession Nos. NM_001134391.1, and NM_031159.3);
APOBEC2: *Homo sapiens* APOBEC2 (protein: GenBank Accession No. NP_006780.1; gene: GenBank Accession No. NM_006789.3), mouse APOBEC2 (protein: GenBank Accession No. NP_033824.1; gene: GenBank Accession No. NM_009694.3);
APOBEC3B: *Homo sapiens* APOBEC3B (proteins: GenBank Accession Nos. NP_001257340.1, and NP_004891.4; genes (genes encoding the proteins previously described are filled in the same order as in the proteins): GenBank Accession Nos. NM_001270411.1, NM_004900.4), mouse (*Mus musculus*) APOBEC3B (protein: GenBank Accession Nos. NP_001153887.1, NP_001333970.1, and NP_084531.1; genes (genes encoding the proteins previously described are filled in the same order as in the proteins): GenBank Accession Nos. NM_001160415.1, NM_001347041.1, and NM_030255.3);
APOBEC3C: *Homo sapiens* APOBEC3C (protein: GenBank Accession No. NP_055323.2; gene: GenBank Accession No. NM_014508.2);
APOBEC3D (including APOBEC3E): *Homo sapiens* APOBEC3D (protein: GenBank Accession No. NP_689639.2; gene: GenBank Accession No. NM_152426.3);
APOBEC3F: *Homo sapiens* APOBEC3F (protein: GenBank Accession Nos. NP_660341.2, and NP_001006667.1; genes (genes encoding the proteins previously described are filled in the same order as in the proteins): NM_145298.5 and NM_001006666.1);
APOBEC3G: *Homo sapiens* APOBEC3G (protein: GenBank Accession Nos. NP_068594.1, NP_001336365.1, NP_001336366.1, and NP_001336367.1; genes (genes encoding the proteins previously described are filled in the same order as in the proteins): NM_021822.3, NM_001349436.1, NM_001349437.1, and NM_00134943 8.1);
APOBEC3H: *Homo sapiens* APOBEC3H (proteins: GenBank Accession Nos. NP_001159474.2, NP_001159475.2, NP_001159476.2, and NP_861438.3; genes (genes encoding the proteins previously described are filled in the same order as in the proteins): NM_001166002.2, NM_001166003.2, NM_001166004.2, and NM_181773.4);
APOBEC4 (including APOBEC3E): *Homo sapiens* APOBEC4 (protein: GenBank Accession No. NP_982279.1; gene: GenBank Accession No. NM_203454.2); mouse APOBEC4 (protein: GenBank Accession No. NP_001074666.1; gene: GenBank Accession No. NM 001081197.1);
Activation-induced cytidine deaminase (AICDA or AID): *Homo sapiens* AID (proteins: GenBank Accession Nos. NP_001317272.1, and NP_065712.1; genes (genes encoding the proteins previously described are filled in the same order as in the proteins): GenBank Accession Nos. NM_001330343.1, and NM_020661.3); mouse AID (protein: GenBank Accession No. NP_033775.1; gene: GenBank Accession No. NM_009645.2); and
CDA (cytidine deaminase; EC number 3.5.4.5; e.g., CDA1): GenBank Accession Nos. NP_001776.1 (gene: NM_001785.2), CAA06460.1 (gene: AJ005261.1), and NP_416648.1 (gene: NC_000913.3).

As used herein, the term "target-specific nuclease", also called programmable nuclease, is intended to encompass all forms of endonucleases that can recognize and cleave specific sites on target genomic DNA.

For example, the target-specific nuclease may be at least one of all the nucleases that has the activity of recognizing and cleaving at specific nucleotide sequences of target genes and thus can cause insertions and/or deletions (Indels) in the target genes.

For example, the target-specific nuclease may be at least selected from the group consisting of, but not limited to, RGENE (RNA-guided engineered nuclease; e.g., Cas9, Cpfl, etc.) derived from the microorganism immune system CRISPR.

According to an embodiment, the target-specific nuclease may be at least one selected from the group consisting of endonucleases included in type I and/or type V of the CRIPR system, such as Cas protein (e.g., Cas9 protein (CRISPR (Clustered regularly interspaced short palindromic repeats) associated protein 9)), Cpfl protein (CRISPR from Prevotella and Francisella 1), etc. In this regard, the target-specific nuclease may further comprise a target DNA-specific guide RNA for guiding to an on-target site in genomic DNA. The guide RNA may be one transcribed *in vitro,* for example, from an oligonucleotide duplex or a plasmid template, but is not limited thereto. The target-specific nuclease and the guide RNA may be used in the form of ribonucleic acid protein (RNP), and the ribonucleic acid protein may be used in a mixture of a target-specific nuclease or a gene coding therefor and a RNA or a gene coding therefor, or in a complex form in which a target-specific nuclease or a gene coding therefor is associated with a RNA or a gene coding therefor.

Cas9 protein is a main protein component of the CRISPR/Cas system, which can function as an activated endonuclease or nickase.

Cas9 protein or gene information thereof may be acquired from a well-known database such as the GenBank of NCBI (National Center for Biotechnology Information). For example, the Cas9 protein may be at least one selected from the group consisting of, but not limited to:
a Cas9 protein derived from *Streptococcus* sp., for example, *Streptococcus pyogenes* (e.g., SwissProt Accession number Q99ZW2(NP_269215.1) (encoding gene: SEQ ID NO: 4);
a Cas9 protein derived from *Campylobacter* sp., for example, *Campylobacter jejuni;*
a Cas9 protein derived from *Streptococcus* sp., for example, *Streptococcus thermophiles* or *Streptocuccus aureus;*
a Cas9 protein derived from *Neisseria meningitidis;*
a Cas9 protein derived from *Pasteurella* sp., for example, *Pasteurella multocida;* and
a Cas9 protein derived from *Francisella* sp., for example, *Francisella novicida.*

Cpfl protein, which is an endonuclease of a new CRISPR system distinguished from the CRISPR/Cas system, is small in size compared to Cas9, requires no tracrRNA, and can function with a single guide RNA. In addition, Cpfl can recognize thymidine-rich PAM (protospacer-adjacent motif) sequences and produces cohesive double-strand breaks (cohesive end).

For example, the Cpfl protein may be an endonuclease derived from *Candidatus* spp., *Lachnospira* spp., *Butyrivibrio* spp., *Peregrinibacteria, Acidominococcus* spp., *Porphyromonas* spp., *Prevotella* spp., *Francisella* spp., *Candidatus Methanoplasma*), or *Eubacterium* spp. Examples of the microorganism from which the Cpfl protien may be derived include, but are not limited to, *Parcubacteria bacterium* (GWC2011_GWC2_44_17), *Lachnospiraceae bacterium* (MC2017), *Butyrivibrio proteoclasiicus, Peregrinibacteria bacterium* (GW2011_GWA_33_10), *Acidaminococcus* sp. (BV3L6), *Porphyromonas macacae, Lachnospiraceae bacterium* (ND2006), *Porphyromonas crevioricanis, Prevotella disiens, Moraxella bovoculi* (237), *Smiihella* sp. (SC_KO8D17), *Leptospira inadai, Lachnospiraceae bacterium* (MA2020), *Francisella novicida* (U112), *Candidatus Methanoplasma termitum, Candidatus Paceibacter,* and *Eubacterium eligens.*

The target-specific endonuclease may be a microorganism-derived protein or an artificial or non-naturally occurring protein obtained by a recombinant or synthesis method. By way of example, the target-specific endonuclease (e.g., Cas9, Cpfl, and the like) may be a recombinant protein produced with a recombinant DNA. As used herein, the term "recombinant DNA (rDNA)" refers to a DNA molecule artificially made by genetic recombination, such as molecular cloning, to include therein heterogenous or homogenous genetic materials derived from various organisms. For instance, when a target-specific endonuclease is produced *in vivo* or *in vitro* by expressing a recombinant DNA in an appropriate organism, the recombinant DNA may have a nucleotide sequence reconstituted with codons selected from among codons encoding the protein of interest in order to be optimal for expression in the organism.

The term "inactivated target-specific endonuclease", as used herein, refers to a target-specific endonuclease that lacks the endonuclease activity of cleaving a DNA duplex. The inactivated target-specific endonuclease may be at least one selected from among inactivated target-specific endonucleases that lack endonuclease activity, but retain nickase activity, and inactivated target-specific endonuclease that lack both endonuclease activity and nickase activity. In an embodiment, the inactivated target-specific endonuclease may retain nickase activity. In this case, when a cytosine base is converted to a uracil base, a nick is introduced into a strand on which cytosine-to-uracil conversion occurs, or an opposite strand thereto simultaneously or sequentially irrespective of order (for example, a nick is introduced at a position between third and fourth nucleotides in the direction toward the 5' end of a PAM sequence on a strand opposite to a strand having the PAM sequence). The modification (mutation) of such target-specific endonucleases may include substitution of a catalytic aspartate residue (for *Streptococcus pyogenes-derived* Cas9 protein, for example, at least one selected from the group consisting of aspartic acid at position 10 (D10), glutamic acid at position 762 (E762), histidine at position 840 (H840), asparagine at position 854 (N854), asparagine at position 863 (N863), and aspartic acid at position 986) with a different amino acid, and the different amino acid may be alanine, but is not limited thereto.

As used herein, the expression "different amino acid" is intended to refer to an amino acid selected from among alanine, isoleucine, leucine, methionine, phenylalanine, proline, tryptophan, valine, asparagine, cysteine, glutamine, glycine, serine, threonine, tyrosine, aspartic acid, glutamic acid, arginine, histidine, lysine, and all known variants thereof, exclusive of the amino acid having a wild-type protein retained at the original substitution position.

In one embodiment, when the inactivated target-specific endonuclease is a modified Cas9 protein, the Cas9 protein may be at least one selected from the group consisting of modified Cas9 that lacks endonuclease activity and retains nickase activity as a result of introducing mutation (for example, substitution with a different amino acid) to D10 or H840 of *Streptococcus pyogenes-derived* Cas9 protein (e.g., SwissProt Accession number Q99ZW2(NP_269215.1)), and modified Cas9 protein that lacks both endonuclease activity and nickase activity as a result of introducing mutations (for example, substitution with different mutations) to both D10 and H840 of *Streptococcus pyogenes-derived* Cas9 protein. In Cas9 protein, for example, the mutation at D10 may be D10A mutation (the amino acid D at position 10 in Cas9 protein is substituted with A; below, mutations introduced to Cas9 are expressed in the same manner), and the mutation at H840 may be H840A mutation. In one embodiment, the inactivated target-specific endonuclease may be a nickase (e.g., encoded by SEQ ID NO: 11) mutated from *Streptococcus pyogenes (Streptococcus pyogenes*)-derived Cas9 protein (SEQ ID NO: 4) by substituting D10 with A (D10A).

The cytidine deaminase and the inactivated target-specific endonuclease may be used in the form of a fusion protein in which they are fused to each other directly or via a peptide linker (for example, existing in the order of cytidine deaminase-inactivated target-specific endonuclease in the N- to C-terminus direction (i.e., inactivated target-specific endonuclease fused to the C-terminus of cytidine deaminase) or in the order of inactivated target-specific endonuclease-cytidine deaminase in the N- to C-terminus direction (i.e., cytidine deaminase fused to the C-terminus of inactivated target-specific endonuclease) (or may be contained in the composition), a mixture of a purified cytidine deaminase or mRNA coding therefor and an inactivated target-specific endonuclease or mRNA coding therefor (or may be contained in the composition), a plasmid carrying both a cytidine deaminase-encoding gene and an inactivated target-specific endonuclease-encoding gene (e.g., the two genes arranged to encode the fusion protein described above) (or may be contained in the composition), or a mixture of a cytidine deaminase expression plasmid and an inactivated target-specific endonuclease expression plasmid which carry a cytidine deaminase-encoding gene and an inactivated target-specific endonuclease-encoding gene, respectively (or may be contained in the composition). In one embodiment, the cytidine deaminase and the inactivated target-specific endonuclease may be in the form of a fusion protein in which they exist in the order of cytidine deaminase-inactivated target-specific endonuclease in the N- to C-terminus direction or in the order of inactivated target-specific endonuclease-cytidine deaminase in the N- to C-terminus direction, or a single plasmid in which a cytidine deaminase-encoding gene and an inactivated target-specific endonuclease-encoding gene are contained to encode the fusion protein.

So long as it carries the cytidine deaminase-encoding gene and/or the inactivated target-specific endonuclease-encoding gene and contains an expression system capable of expressing the gene in a host cell, any plasmid may be used. The plasmid contains elements for expressing a target gene, which include a replication origin, a promoter, an operator, and a terminator, and may further comprise an enzyme site suitable for introduction into the genome of a host cell (e.g., restriction enzyme site), a selection marker for identifying successful introduction into a host cell, a ribosome binding site (RBS) for translation into a protein, and/or a transcriptional regulatory factor. The plasmid may be one used in the art, for example, at least one selected from the group consisting of, but not limited to, pcDNA series, pSC101, pGV1106, pACYC177, ColE1, pKT230, pME290, pBR322, pUC8/9, pUC6, pBD9, pHC79, pIJ61, pLAFR1, pHV14, pGEX series, pET series, and pUC19. The host cell may be selected from among cells to which base editing or a double-strand break is intended to introduced by the cytidine deaminase (for example, eukaryotic cells including mammal cells such as human cells) and all cells that can express the cytidine deaminase-encoding gene and/or the inactivated target-specific endonuclease-encoding gene into cytidine deaminase and inactivated target-specific endonuclease, respectively (for example, E. coli, etc.).

The guide RNA, which acts to guide a mixture or a fusion protein of the cytidine deaminase and the inactivated target-specific endonuclease to an on-target site, may be at least one selected from the group consisting of CRISPR RNA (crRNA), *trans*-activating crRNA (tracrRNA), and single guide RNA (sgRNA), and may be, in detail, a crRNA:tracrRNA duplex in which crRNA and tracrRNA is coupled to each other, or a single-strand guide RNA (sgRNA) in which crRNA or a part thereof is connected to tracrRNA or a part thereof via an oligonucleotide linker.

Concrete sequences of the guide RNA may be appropriately selected, depending on kinds of the target-specific endonucleases used, or origin microorganisms thereof, and are an optional matter which could easily be understood by a person skilled in the art.

When a *Streptococcus pyogenes*-derived Cas9 protein is used as a target-specific endonuclease, crRNA may be represented by the following General Formula 1:
5'-(N_{cas9})ₗ-(GUUUUAGAGCUA)-(X_{cas9})ₘ-3' (General Formula 1)
wherein,
N_{cas9} is a targeting sequence, that is, a region determined according to a sequence at an on-target site in a target gene (i.e., a sequence hybridizable with a sequence of an on-target site), 1 represents a number of nucleotides included in the targeting sequence and is an integer of 17 to 23 or 18 to 22, for example, 20;
the region including 12 consecutive nucleotides (GUUUUAGAGCUA; SEQ ID NO: 1) adjacent to the 3'-terminus of the targeting sequence is essential for crRNA,
X_{cas9} is a region including m nucleotides present at the 3'-terminal site of crRNA (that is, present adjacent to the 3'-terminus of the essential region), and m may be an integer of 8 to 12, for example, 11 wherein the m nucleotides may be the same or different and are independently selected from the group consisting of A, U, C, and G.

In an embodiment, the X_{cas9} may include, but is not limited to, UGCUGUUUUG (SEQ ID NO: 2).

In addition, the tracrRNA may be represented by the following General Formula 2:
5'-(Y_{cas9})ₚ-(UAGCAAGUUAAAAUAAGGCUAGUCCGUUAUCAACUUGAAAAAGUGGCACCGAGUCGGUGC)-3' (General Formula2)
wherein,
the region represented by 60 nucleotides (UAGCAAGUUAAAAUAAGGCUAGUCCGUUAUCAACUUGAAAAAGUGGCACCGAGUCGGUGC; SEQ ID NO: 3) is essential for tracrRNA,

Y_{cas9} is a region including p nucleotides present adjacent to the 3'-terminus of the essential region, and p may be an integer of 6 to 20, for example, 8 to 19 wherein the p nucleotides may be the same or different and are independently selected from the group consisting of A, U, C, and G.

Further, sgRNA may form a hairpin structure (stem-loop structure) in which a crRNA moiety including the targeting sequence and the essential region thereof and a tracrRNA moiety including the essential region (60 nucleotides) thereof are connected to each other via an oligonucleotide linker (responsible for the loop structure). In greater detail, the sgRNA may have a hairpin structure in which a crRNA moiety including the targeting sequence and essential region thereof is coupled with the tracrRNA moiety including the essential region thereof to form a double-strand RNA molecule with connection between the 3' end of the crRNA moiety and the 5' end of the tracrRNA moiety via an oligonucleotide linker.

In one embodiment, sgRNA may be represented by the following General Formula 3:
5'-(N_{cas9})ₗ-(GUUUUAGAGCUA)-(oligonucleotide linker)-(UAGCAAGUUAAAAUAAGGCUAGUCCGUUAUCAACUUGAAAAAGUGGCACCGAGUCGGUGC)-3' (General Formula 3)
wherein, (N_{cas9})ₗ is a targeting sequence defined as in General Formula 1.

The oligonucleotide linker included in the sgRNA may be 3-5 nucleotides long, for example 4 nucleotides long in which the nucleotides may be the same or different and are independently selected from the group consisting of A, U, C, and G.

The crRNA or sgRNA may further contain 1 to 3 guanines (G) at the 5' end thereof (that is, the 5' end of the targeting sequence of crRNA).

The tracrRNA or sgRNA may further comprise a terminator inclusive of 5 to 7 uracil (U) residues at the 3' end of the essential region (60 nt long) of tracrRNA.

The target sequence for the guide RNA may be about 17 to about 23 or about 18 to about 22, for example, 20 consecutive nucleotides adjacent to the 5' end of PAM (Protospacer Adjacent Motif (for *S. pyogenes* Cas9, 5'-NGG-3' (N is A, T, G, or C)) on a target DNA.

As used herein, the term "the targeting sequence" of guide RNA hybridizable with the target sequence for the guide RNA refers to a nucleotide sequence having a sequence complementarity of 50 % or higher, 60 % or higher, 70 % or higher, 80 % or higher, 90 % or higher, 95 % or higher, 99 % or higher, or 100 % to a nucleotide sequence of a complementary strand to a DNA strand on which the target sequence exists (i.e., a DNA strand having a PAM sequence (5'-NGG-3' (N is A, T, G, or C))) and thus can complimentarily couple with a nucleotide sequence of the complementary strand.

In the present specification, a nucleic acid sequence at an on-target site is represented by that of the strand on which a PAM sequence exists among two DNA strands in a region of a target gene. In this regard, the DNA strand to which the guide RNA couples is complementary to a strand on which a PAM sequence exists. Hence, the targeting sequence included in the guide RNA has the same nucleic acid sequence as a sequence at an on-target site, with the exception that U is employed instead of T due to the RNA property. In other words, a targeting sequence of guide RNA and a sequence at the on-target site (or a sequence of a cleavage site) are represented by the same nucleic acid sequence with the exception that T and U are interchanged, in the present specification.

The guide RNA may be used in the form of RNA (or may be contained in the composition) or in the form of a plasmid carrying a DNA coding for the RNA (or may be contained in the composition).

The composition and method described in the present specification may comprise or may not use a Uracil-Specific Excision Reagent (USER). The term "uracil-specific excision reagent", as used herein, is intended to encompass any material that plays a role in excising uracil residues converted from cytosine residues by the cytidine deaminase and/or inducing DNA cleavage at the uracil-excised positions.

According to an embodiment, the uracil-specific excision reagent (USER) includes uracil DNA glycosylase (UDG), endonuclease VIII, or a combination thereof. In one embodiment, the uracil-specific excision reagent may comprise endonuclease VIII or a combination of uracil DNA glycosylase and endonuclease VIII.

Uracil DNA glycosylase (UDG) is an enzyme that functions to prevent mutagenesis eliminating uracil from DNA molecules and may be at least one selected from all enzymes that play a role in cleaving the N-glycosylic bond to initiate the base-excision repair (BER) pathway. By way of example, the uracil DNA glycosylase may be at least one selected from the group consisting of, but not limited to, *Escherichia coli* uracil DNA glycosylases (e.g., GenBank Accession Nos. ADX49788.1, ACT28166.1, EFN36865.1, BAA10923.1, ACA76764.1, ACX38762.1, EFU59768.1, EFU53885.1, EFJ57281.1, EFU47398.1, EFK71412.1, EFJ92376.1, EFJ79936.1, EFO59084.1, EFK47562.1, KXH01728.1, ESE25979.1, ESD99489.1, ESD73882.1, ESD69341.1, etc.), human uracil DNA glycosylases (e.g., GenBank Accession Nos. NP_003353.1, NP_550433.1, etc.), and mouse uracil DNA glycosylases (e.g., GenBank Accession Nos. NP_001035781.1, NP_035807.2, etc.).

Endonuclease VIII acts to excise damaged uracil residues from double-stranded DNA while eliminating the uracil-excised nucleotides and may be at least one selected from among all enzymes that have N-glycosylase activity of releasing the uracil residues damaged by uracil DNA glycosylase, generating an apurinic site (AP-site) and AP-lyase activity of cleaving 3' and 5' to the AP site. For example, the endonuclease VIII may be at least one selected from the group consisting of human endonuclease VIII (e.g., GenBank Accession Nos. BAC06476.1, NP_001339449.1, NP_001243481.1, NP_078884.2, NP_001339448.1, etc.), mouse endonuclease VIII (e.g., GenBank Accession Nos. BAC06477.1, NP_082623.1, etc.), and *Escherichia coli* endonuclease VIII (e.g., GenBank Accession Nos. OBZ49008.1, OBZ43214.1, OBZ42025.1, ANJ41661.1, KYL40995.1, KMV55034.1, KMV53379.1, KMV50038.1, KMV40847.1, AQW72152.1, etc.), but is not limited thereto.

Another aspect provides a method for inducing a double-strand break in DNA, the method comprising a step of introducing into a cell or contacting with DNA separated from cells, (a) a deaminaseor a gene coding therefor (cDNA, rDNA, or mRNA), (b) an inactivated target-specific endonuclease or a gene coding therefor (cDNA, rDNA, or mRNA), and (c) a guide RNA or a gene coding therefor. This method may not comprise a step of treating with a uracil-specific excision reagent (USER).

As such, the production (or introduction) of a single-strand break in DNA allows for analyzing sites of genomic DNA or on-target sites of DNA in which cytidine deaminase makes base editing (conversion from C to U) or produces (introduces) the single-strand break, and for base editing efficiency, whereby base editing efficiency in on-target sites, specificity for on-target sequences, and off-target sequences can be identified (or measured).

Another aspect provides a method for analyzing a nucleic acid sequence of DNA to which base editing is introduced by deaminase, the method comprising the steps of:
(i) introducing into a cell or contacting with DNA separated from cells (a) a deaminaseor a gene coding therefor (cDNA, rDNA, or mRNA), (b) an inactivated target-specific endonuclease or a gene coding therefor(cDNA, rDNA, or mRNA), and (c) a guide RNA or a gene coding therefor to induce a single-strand break in the DNA; and
(ii) analyzing a nucleic acid sequence of a DNA fragment in which the single-strand break has been induced. The method may not comprise a step of treating with a uracil-specific excision reagent (USER) to produce a double-strand break in DNA.

Another aspect provides a method for identifying (or measuring or detecting) a base-editing site, a single-strand break site, base editing efficiency at an on-target site, an off-target site, and target specificity of deaminase, the method comprising the steps of:
(i) introducing to a cell or contacting with DNA separated from cells (a) a deaminaseor a gene coding therefor (cDNA, rDNA, or mRNA), (b) an inactivated target-specific endonuclease or a gene coding therefor(cDNA, rDNA, or mRNA), and (c) a guide RNA or a gene coding therefor to induce a single-strand break in DNA;
(ii) analyzing a nucleic acid sequence of the cleaved DNA fragment; and
(iii) identifying a single-strand break site from the nucleic acid sequence data obtained by the analysis. The method may further comprise, for example, the step of (iii-1) identifying base editing (e.g., conversion of cytosine (C) to uracil (U) or thymine (T)) in the nucleic acid sequence data obtained by the analysis (sequence read) between steps (ii) and (iii) or concomitantly with, prior to or after step (iii). The method may not comprise a step of treating with a uracil-specific excision reagent (USER) to induce a double-strand break in DNA.

In one embodiment, the method (for identifying, for example, base editing efficiency at an on-target site, and an off-target site) may further comprise, after step (iii), a step of (iv) identifying (determining) the break position as an off-target site when the break position is not within an on-target site.

The deaminase, the inactivated target-specific endonuclease, the guide RNA, and the uracil-specific excision reagent are as defined above.

The methods provided in the present specification may be conducted in cells (which may be separated from a living body) or *in vitro* (extracellularly). For example, the methods may be executed *in vitro* (extracellularly). In greater detail, all the steps of the methods may be conducted *in vitro.* Alternatively, step (i) may be conducted in cells while step (ii) and subsequent steps may be conducted *in vitro* (extracellularly) with the DNA (e.g., genomic DNA) extracted from the cells in which step (i) has been conducted.

In step (i), a deaminase (or a gene coding therefor), an inactivated target-specific endonuclease (or a gene coding therefor), and a guide RNA are transfected into cells or are contacted (e.g., incubated) with DNA extracted from cells to induce base editing (base conversion, e.g., from cytosine to uracil) at an on-target site targeted by the guide RNA and the generation of nicks in a single strand of the DNA. The cells may be selected from among all eukaryotic cells to which base editing and/or single-strand breaks by deaminase are to be introduced, and from among, for example, mammal cells including human cells.

The transfection may be performed using any typical method for introducing to cells
(1) a mixture of a deaminase, an inactivated target-specific endonuclease, and a guide RNA or a complex in which they are associated with one another (ribonucleic acid protein; RNP),
(2) a mixture of a deaminase-encoding mRNA, an inactivated target-specific endonuclease-encoding mRNA, and a guide RNA,
(3) a plasmid (recombinant vector) carrying both a deaminase-encoding gene and a target-specific endonuclease-encoding gene or plasmids (recombinant vectors) respectively carrying a deaminase-encoding gene and a target-specific endonuclease-encoding gene, and a guide RNA or a plasmid carrying a guide RNA-encoding gene. By way of example, the introduction may be conducted by electroporation, lipofection, microinjection, etc., but is not limited thereto.

In one embodiment, the step (i) may be carried out by incubating a DNA extracted from cells (which is to be identified for the base editing (base-editing site, base editing efficiency, etc.) and/or single-strand break (cleavage positions, cleavage efficiency, etc.) by a deaminase and an inactivated endonuclease) with a deaminase and an inactivated target-specific endonuclease (e.g., a fusion protein containing both a cytidine deaminase and an inactivated Cas9 protein), and a guide RNA (*in vitro*). The DNA extracted from cells may be a genome DNA, a target gene, or a PCR (polymerase chain reaction) product inclusive of the target gene.

Optionally, a step of removing the deaminase, the inactivated target-specific endonuclease, and/or the guide RNA, all used in step (i), may be further comprised after step (i) and before step (ii). In addition, the method may further comprise a step of making blunt (or repairing) an end of the double-strand DNA fragment in which a single-strand break has been generated, after step (i) and before step (ii). The step of making an end blunt may include (b) a 3'-to-5' trimming step in which elimination (excision) is made of the overhangs at the 3'end of the uncleaved strand of the double-strand DNA fragment where a single-strand break has been induced and/or (c) a 5'-to-3' DNA synthesis step in which extension is made of the 3'-terminal nucleotide from the break point of the cleaved strand of the double-strand DNA fragment where a single-strand break has been induced (see diagram in Example 1). The 3'-to-5' trimming step may be carried out using a suitable typical exonuclease. The 5'-to-3' DNA synthesis step may be carried out using a suitable typical DNA polymerase.

Optionally, the method may further comprise, after step (i) and before step (ii), a step of amplifying the DNA fragment in which a single-strand break has been induced (of the DNA duplex, an oligonucleotide composed of 10 to 30 or 15 to 25 consecutive nucleotides inclusive of the cleavage site of the cleaved strand and/or an oligonucleotide composed of 10 to 30 or 15 to 25 consecutive nucleotides, corresponding (complementary) to the cleavage site, of the uncleaved strand in order to facilitate the nucleic acid sequence analysis of the DNA fragment in step (ii). For use in analysis in step (ii), the DNA fragment where a single-strand break has been induced may comprise an oligonucleotide composed of 10 to 30 or 15 to 25 consecutive nucleotides inclusive of the cleavage site of the cleaved strand and/or an oligonucleotide composed of 10 to 30 or 15 to 25 consecutive nucleotides, corresponding (complementary) to the cleavage site, of the uncleaved strand; and/or an amplification product of the oligonucleotide.

Thanks to being used together with the guide RNA, the deaminase and the inactivated target-specific endonuclease show sequence specificity and, for the most part, act on target sites (on-target). Depending on the extent to which sequences similar to the target sequence exist in sites except the on-target site, however, the side effect of acting on off-target sites may occur. As used herein, the term "off-target site" refers to a site which is not the on-target site of the deaminase and the inactivated target-specific endonuclease, but allows he deaminase and the inactivated target-specific endonuclease to be active therein, that is, a site, except the on-target site, in which base editing and/or cleavage is induced by the deaminase and the inactivated target-specific endonuclease. In one embodiment, the off-target site is intended to encompass not only actual off-target sites but also potential sites which are likely to be off-target sites. The off-target site may include, but is not limited to, all sites, except the on-target site cleaved *in vitro* by the deaminase and the inactivated target-specific endonuclease.

There are various causes that make the deaminase and the inactivated target-specific endonuclease be active in sites except the on-target site. For example, the deaminase and the inactivated target-specific endonuclease may be apt to work for non-target sequences (off-target sequences) which have high sequence homology to a target sequence due to a low level of nucleotide mismatch with the target sequence designed for an on-target site.

The off-target site may be a sequence site (gene region) that satisfies at least one of the following conditions:
The number of DNA reads of which the 5' ends are vertically aligned is 2 or greater, for example, 3 or greater, 4 or greater, 5 or greater, 6 or greater, 7 or greater, 8 or greater, 9 or greater, or 10 or greater;
A strand complementary to the strand on which a break has been induced in a double-stranded DNA fragment includes a PAM sequence;
A complementary strand to the strand on which a break has been induced in a double-stranded DNA fragment includes 15 or less or 10 or less nucleotide mismatches with a sequence at the on-target site (target sequence), for example, 1 to 15, 1 to 14, 1 to 13, 1 to 12, 1 to 11, 1 to 10, 1 to 9, 1 to 8, 1 to 7, 1 to 6, 1 to 5, 1 to 4, 1 to 3, 1 to 2, or 1 nucleotide mismatches; and
A complementary strand to the strand on which a break has been induced in a double-stranded DNA fragment includes base editing (conversion of at least cytosine (C) residue to uracil (U) or thymine (T)).

The working of the deaminase and the inactivated target-specific endonuclease in an off-target site may incur undesirable mutation in a genome, which may lead to a significant problem. Hence, a process of accurately detecting and analyzing an off-site sequence may be as very important as the activity of the deaminase and the inactivated target-specific endonuclease at an on-target site. The process may be useful for developing a deaminase and an inactivated target-specific endonuclease which both work specifically only at on-target sites without the off-target effect.

Because the cytidine deaminase and the inactivated target-specific endonuclease have activities *in vivo* and *in vitro* for the purpose of the present invention, the enzymes can be used in detecting *in vitro* an off-target site of DNA (e.g., genomic DNA). When applied *in vivo,* thus, the enzymes are expected to be active in the same sites (gene loci including off-target sequences) as the detected off-target sites.

The step (ii) in which a nucleic acid sequence of the DNA fragment cleaved (single-strand breaks) in step (i) is analyzed may be carried out using any typical nucleic acid analysis method. For example, when the separate DNA used in step (i) is a genomic DNA, the nucleic acid sequence analysis may be conducted by whole genome sequencing. In contrast to the indirect method in which a sequence having a homology with the sequence at an on-target site is searched for and would be predicted to be off-target site, whole genome sequencing allows for detecting an off-target site actually cleaved by the target-specific nuclease at the level of the entire genome, thereby more accurately detecting an off-target site.

As used herein, the term "whole genome sequencing" (WGS) refers to a method of reading the genome by many multiples such as in 10 X, 20 X, and 40 X formats for whole genome sequencing by next generation sequencing. The term "Next generation sequencing" means a technology that fragments the whole genome or targeted regions of genome in a chip-based and PCR-based paired end format and performs sequencing of the fragments by high throughput on the basis of chemical reaction (hybridization).

In the step (iii), a DNA cleavage site is identified (or determined) using the base sequence data (sequence read) obtained in step (ii). By analyzing sequencing data, an on-target site and an off-target site can simply be detected. The determination of a site at which DNA is cleaved from the base sequence data can be performed by various approaches. In the specification, various reasonable methods are provided for determining the site. However, they are merely illustrative examples that fall within the technical spirit of the present invention, but are not intended to limit the scope of the present invention.

As an example of determining a cleaved site, when the sequence reads obtained by whole genome sequencing are aligned according to sites on a genome, the site at which the 5' ends are vertically aligned may mean the site at which DNA is cleaved. The alignment of the sequence reads according to sites on genomes may be performed using an analysis program (for example, BWA/GATK or ISAAC). As used herein, the term "vertical alignment" refers to an arrangement in which the 5' ends of two more sequence reads start at the same site (nucleotide position) on the genome for each of the adjacent Watson strand and Crick strand when the whole genome sequencing results are analyzed with a program such as BWA/GATK or ISAA. Through this method, the DNA fragments that are cleaved in step (ii) and thus have the same 5' end are each sequenced.

That is, when the cleavage in step (i) occurs at on-target sites and off-target sites, the alignment of the sequence reads allows the vertical alignment of the common cleaved sites because each of their sites start at the 5' end. However, the 5' end is not present in the uncleaved sites, so that it can be arranged in a staggered manner in alignment. Accordingly, the vertically aligned site may be regarded as a site cleaved in step (i), which means an on-target site or off-target site cleaved by the inactivated target-specific endonuclease.

The term "alignment" means mapping sequence reads to a reference genome and then aligning the bases having identical sites in genomes to fit for each site. Accordingly, so long as it can align sequence reads in the same manner as above, any computer program may be employed. The program may be one already known in the pertinent art or may be selected from among programs tailored to the purpose. In one embodiment, alignment is performed using ISAAC, but is not limited thereto.

As a result of the alignment, the site at which the DNA is cleaved by the deaminase and the inactivated target-specific endonuclease can be determined by a method such as finding a site where the 5' end is vertically aligned as described above, and the cleaved site may be determined as an off-target site if not an on-target site. In other words, a sequence is an on-target site if identical to the base sequence designed as an on-target site of the deaminase and inactivated target-specific endonuclease, and is regarded as an off-target site if not identical to the base sequence. This is obvious according to the definition of an off-target site described above.

The method (e.g., method for identifying base editing efficiency at an on-target site and determining an off-target site) may further include a step of identifying (determining) the cleavage site to be an off-target site if the cleavage site is not an on-target site, after step (iii).

The cleaved strands of DNA fragments cleaved by a base editor (deaminase and inactivated target-specific endonuclease) may have 5' ends vertically aligned. According to the number of DNA read(s) with 5' ends vertically aligned (as used herein, term "DNA read(s)" refers to a DNA fragment or a set of DNA fragments which have 5' ends vertically aligned and the same nucleic acid sequence), the number of cleavage sites can be identified. For example, when the number of a DNA read is 1, cleavage by the base editor can be determined to occur only at one site, that is, the on-target site. When the number of the DNA reads of which the 5' ends are vertically aligned is 2 or greater, for example, 3 or greater, 4 or greater, 5 or greater, 6 or greater, 7 or greater, 8 or greater, 9 or greater, or 10 or greater, cleavage occurs at two or more sites, indicating that DNA was cleaved at at least one site which is not an on-target site (off-target site). DNA reads the 5' ends of which are vertically aligned can be identified (or determined) to be off-target sites if they are not an on-target site (that is, have nucleic acid sequences different from that of the on-target site).

Therefore, the step (iii) of identifying a site at which the single-strand is cleaved may comprise (a) identifying (or measuring) a number of DNA reads. In this regard, when the number of DNA reads the 5' ends of which are vertically aligned are 2 or greater, for example, 3 or greater, 4 or greater, 5 or greater, 6 or greater, 7 or greater, 8 or greater, 9 or greater, or 10 or greater, DNA cleavage can be identified (or determined) to occur at one or more non-target sites (off-target sites). In this case, in addition, the step (iv) of determining an off-target site may comprise a step of (iv-1) identifying (or determining) as an off-target site at least one of two or more DNA reads of which the 5' ends are vertically aligned if the one has a nucleic acid sequence different from that of the on-target site.

Furthermore, determining whether the off-target site includes a PAM sequence (in greater detail, whether a PAM sequence is included in a complementary strand (strand having a complementary sequence) to a DNA read of which the 5' end is vertically aligned and which has a nucleic acid different from that of an on-target site) can exclude a site at which cleavage has been made by error, but not by the target-specific endonuclease included in the base editor, thereby further increasing accuracy for off-target sites. Thus, the step (iii) of identifying a site at which a single-strand break has been induced may further comprise a step of (b) determining whether the off-target site includes a PAM sequence, for example, whether a PAM sequence specific for the target-specific endonuclease of the base editor is included in a complementary strand (a strand having a complementary sequence) to a DNA read of cleaved DNA fragments of which the 5' end is vertically aligned and which has a nucleic acid sequence different from that of the on-target site. In this regard, the step (iv) of identification as an off-target site may comprise a step of (iv-2) identifying (or determining), as an off-target site, a DNA read of cleaved DNA fragment of which the 5' end is vertically aligned and which has a nucleic acid sequence different from that of the on-target site when the DNA read includes a PAM sequence specific for the target-specific endonuclease of the base editor.

In addition, the off-target site may be composed of a sequence having a homology to the sequence of an on-target site. More specifically, because a sequence at an on-target site is represented by a nucleic acid sequence on a strand including a PAM sequence, a sequence at an off-target site may be a nucleic acid sequence of a complementary strand (a strand having a complementary sequence) to a DNA read of cleaved DNA fragments of which the 5' end is vertically aligned and which is different in nucleic acid sequence from the on-target site. In this context, the sequence at on off-target site may have one or more nucleotide mismatches with the sequence at the on-target site, more particularly, 15 or less or 10 or less, for example, 1 to 15, 1 to 14, 1 to 13, 1 to 12, 1 to 11, 1 to 10, 1 to 9, 1 to 8, 1 to 7, 1 to 6, 1 to 5, 1 to 4, 1 to 3, or 1 to 2 nucleotide mismatches.

Hence, the step (iii) of identifying a site at which a single-strand break has been induced may further comprise a step of (c) identifying (or measuring) a number of nucleotide mismatches between a complementary strand and a sequence at an on-target site, the complementary strand having a sequence complementary to a DNA read of cleaved DNA fragments of which the 5' end is vertically aligned and which has a nucleic acid sequence different from that at the on-target site. When the number of the nucleotide mismatches is 15 or less or 10 or less, for example, 1 to 15, 1 to 14, 1 to 13, 1 to 12, 1 to 11, 1 to 10, 1 to 9, 1 to 8, 1 to 7, 1 to 6, 1 to 5, 1 to 4, 1 to 3, or 1 to 2, the occurrence of DNA cleavage at an off-target site can be identified (or determined). In this regard, the step (iv) of identifying as an off-target site may comprise a step of (iv-3) identifying (or determining0 as an off-target site when a complementary strand (a strand having a complementary sequence) to a DNA read of cleaved DNA fragments of which the 5' end is vertically aligned and which is different in nucleic acid sequence from the on-target site has 15 or fewer or 10 or fewer nucleotide mismatches with the sequence at the on-target site, for example, 1 to 15, 1 to 14, 1 to 13, 1 to 12, 1 to 11, 1 to 10, 1 to 9, 1 to 8, 1 to 7, 1 to 6, 1 to 5, 1 to 4, 1 to 3, or 1 to 2 nucleotide mismatches.

The step (iii) may include at least one of steps (a), (b), and (c) (for example, step (a) and at least one of steps (b) and (c)). When two or more of steps (a), (b), and (c) are included, they may be conducted at the same time or irrespective of the order thereof. In addition, the step (iv) may include at least of steps (iv-1), (iv-2), and (iv-3) (for example step (iv-1) and at least one of steps (iv-2) and (iv-3)). When two or more of steps (iv-1), (iv-2), and (iv-3) are included, they may be conducted at the same time or irrespective of the order thereof.

The step (iii-1) of identifying whether base editing (e.g., conversion of cytosine (C) to uracil (U) or thymine (T)) is induced may include a step of identifying (determining) whether a nucleic acid sequence of a complementary strand (a strand having a complementary sequence) to a DNA read of cleaved DNA fragments of which the 5' end is vertically aligned and which is different in nucleic acid sequence from the on-target site contains base editing (conversion of at least one cytosine (C) residue to a uracil (U) or thymine (T) residue). In this case, the step (iv) of identifying as an off-target site 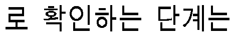 , a step of (iv-4) identifying as an off-target site when a sequence at an off-target site may be a nucleic acid sequence of a complementary strand (a strand having a complementary sequence) to a DNA read of cleaved DNA fragments of which the 5' end is vertically aligned and which is different in nucleic acid sequence from the on-target site contains base editing (conversion of at least one cytosine (C) residue to a uracil (U) or thymine (T) residue).

In an embodiment, the step (i) is conducted with regard to the genomic DNA to induce a single-strand break and after the whole genome analysis (step (ii), the DNA reads are aligned with ISAAC to identify alignment patterns for vertical alignment at cleaved sites and staggered alignment at uncleaved sites. A unique pattern may appear at the cleavage sites as represented by a 5' end plot.

Moreover, as a non-limiting examples, the site where two or more sequence reads corresponding to Watson strand and Crick strand are aligned vertically may be determined as an off-target site. In addition, the site where 20% or more of sequence reads are vertically aligned and the number of sequence reads having the same 5' end in each of the Watson and Creek strands is 10 or more is determined as an off-target site position, that is, a cleavage site.

The process in steps (ii) and (iii) of the method described above may be Digenome-seq (digested-genome sequencing). For greater details, reference may be made to Korean Patent No. 10-2016-0058703 A (this document is herein incorporated by reference in its entirety).

Base editing sites and/or single-strand break sites of the deaminase, base editing efficiency at on-target sites or target specificity (i.e., [base editing or cleavage frequency at on-target sites]/[base editing or cleavage frequency over entire sequence]), and/or off-target sites (identified as base editing sites of deaminase, but not on-target sites) can be identified (or measure or detected) by the method described above.

The identification (detection) of an off-target site is performed *in vitro* by treating a genomic DNA with the deaminase and the inactivated target-specific endonuclease. Thus, it can be identified whether off-target effects are actually produced also *in vivo* in the off-target site detected by this method. However, this is merely an additional verification process, and thus is not a step that is essentially entailed by the scope of the present invention, and is merely a step that can be additionally performed according to the needs.

In the present specification, the term "off-target effect" is intended to mean a level at which base editing and/or double-strand break occurs at an off-target site. The term "indel" (insertion and/or deletion) is a generic term for a mutation in which some bases are inserted or deleted in the middle of a base sequence of DNA.

### EFFECT OF THE INVENTION

The method for inducing a single-strand break in DNA, using a cytidine deaminase and the nucleic acid sequence analysis technique using the same, both provided in the present specification, can more accurately and effectively identify base editing sites, target specificity, and/or off-target sites of the cytidine deaminase.

### BRIEF DESCRIPTION OF THE DRAWINGS

FIG. 1 is a representative IGV image showing straight alignments of sequence reads at the EMX1 on-target site.
FIG. 2 shows the number of nicked sites at which sequence reads have uniform alignment only in one strand obtained as a result of the Digenome-seq (sites (reads) at which the 5' ends have straight alignment) and the number of PAM-containing sites with 10 or fewer mismatches among the sites.
FIG. 3a is a cleavage map of the rAPOBEC1-XTEN-dCas9-NLS vector.
FIG. 3b is a cleavage map of the rAPOBEC1-XTEN-dCas9-UGI-NLS vector.
FIG. 3c is a cleavage map of the rAPOBEC1-XTEN-Cas9n-UGI-NLS vector.
FIG. 4 is a cleavage map of the Cas9 expression plasmid vector.
FIG. 5 is a cleavage map of the pET28b-BE1 vector.
FIG. 6 is a cleavage map of the pET28b-BE3 delta UGI vector.

### EXAMPLES

Hereafter, the present invention will be described in detail by examples.

The following examples are intended merely to illustrate the invention and are not construed to restrict the invention.

### [REFERENCE EXAMPLE]

### 1. Cell culture and Transfection

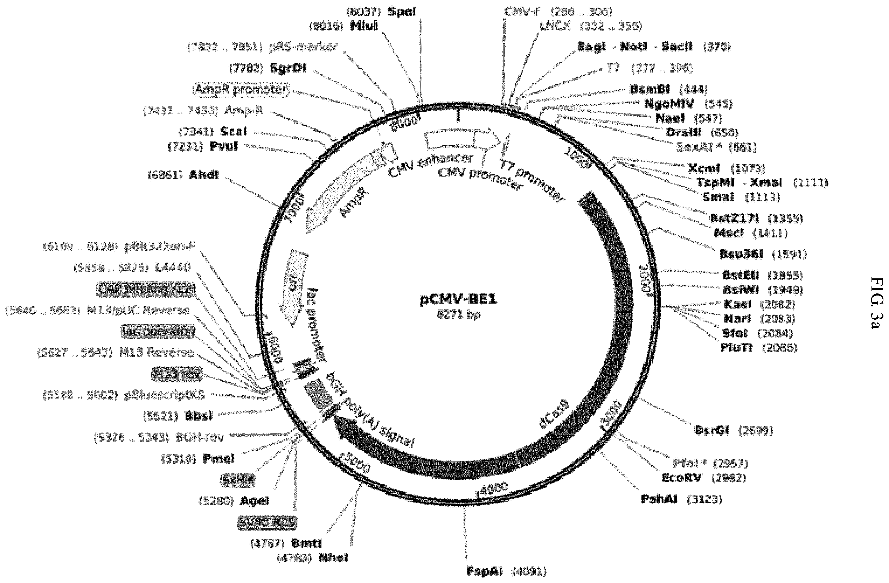

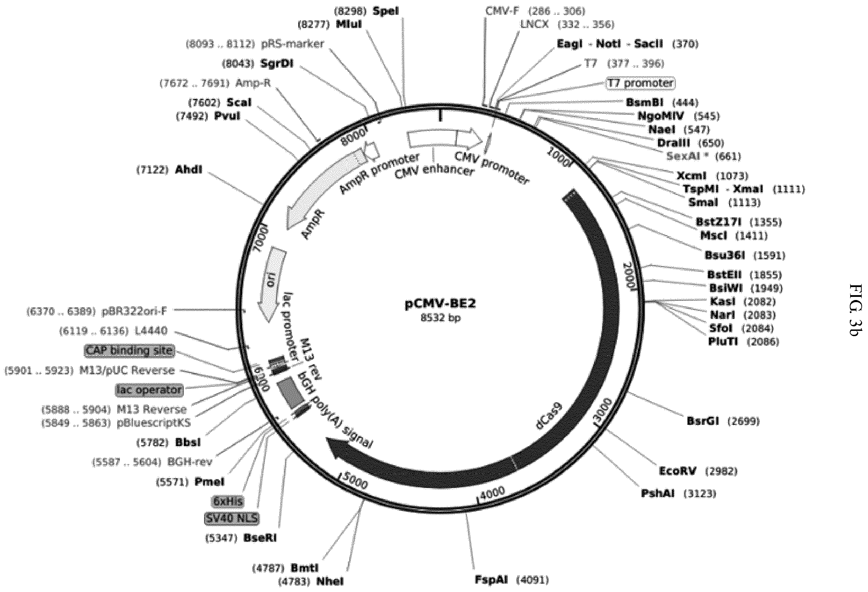

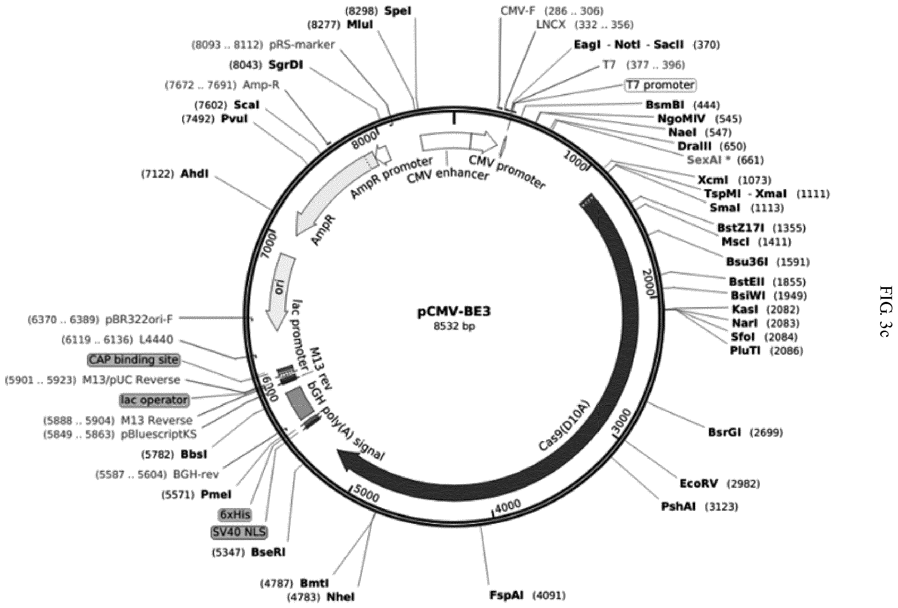

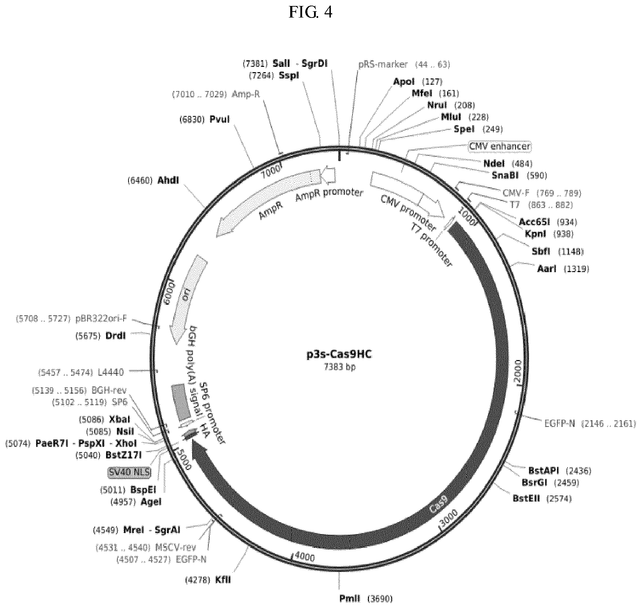

HEK293T cells (ATCC CRL-11268) were maintained in DMEM (Dulbecco Modified Eagle Medium) supplemented with 10% (w/v) FBS and 1% (w/v) penicillin/streptomycin (Welgene). HEK293T cells (1.5 x 10⁵) were seeded on 24-well plates and transfected at ∼80% confluency with sgRNA plasmid (500 ng) and Base Editor plasmid (Addgene plasmid #73019 (Expresses BE1 with C-terminal NLS in mammalian cells; rAPOBEC1-XTEN-dCas9-NLS; FIG. 3a), #73020 (Expresses BE2 in mammalian cells; rAPOBEC1-XTEN-dCas9-UGI-NLS; FIG. 3b), #73021 (Expresses BE3 in mammalian cells; rAPOBEC1-XTEN-Cas9n-UGI-NLS; FIG. 3c)) (1.5µg) or Cas9 expression plasmid (Addgene plasmid #43945; FIG. 4), using Lipofectamine 2000 (Invitrogen). Genomic DNA was isolated using DNeasy Blood & Tissue Kit (Qiagen) at 72 hours after transfection. The cells were not tested for mycoplasma contamination.

The sgRNA used in the following Examples was constructed by converting T to U on the overall sequence at an on-target site (on-target sequence; EMX1 on-target sequence; GAGTCCGAGCAGAAGAAGAAGGG (SEQ ID NO: 14)), except the 5'-terminal PAM sequence ((5'-NGG-3') wherein N is A, T, G, or C), and employing the converted sequence as the targeting sequence '(N_{cas9})ₗ' of the following General Formula 3:
5'-(N_{cas9})ₗ-(GUUUUAGAGCUA; SEQ ID NO: 1)-(GAAA)-(UAGCAAGUUAAAAUAAGGCUAGUCCGUUAUCAACUUGAAAAAGUGGCAC CGAGUCGGUGC; SEQ ID NO: 3)-3' (General Formula 3; oligonucleotide linker: GAAA).

### 2. Protein purification

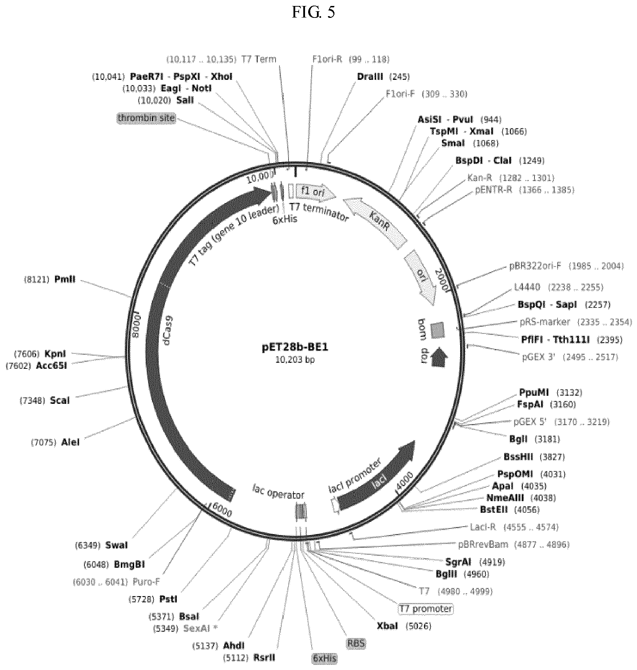

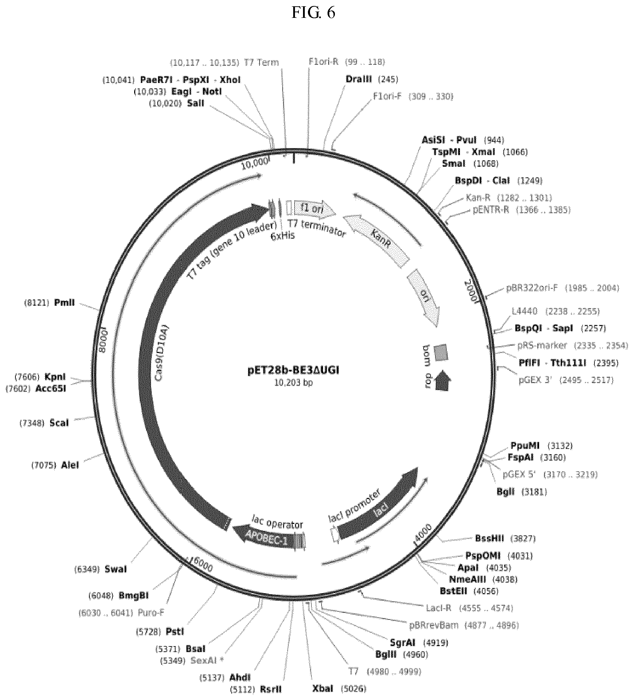

The His6-rAPOBEC1-XTEN-dCas9 protein-encoding plasmid (pET28b-BE1; Expresses BE1 with N-terminal His6 tag in E. coli; FIG. 5) was generously given by David Liu (Addgene plasmid #73018). The His6-rAPOBEC1-XTEN-dCas9 protein-encoding plasmid pET28b-BE1 was converted into a His6-rAPOBEC1-nCas9 protein (BE3 delta UGI; BE3 variant lacking a UGI domain) encoding plasmid (pET28b-BE3 delta UGI; FIG. 6) by site directed mutagenesis for substituting A840 with H840 in the dCas9.

Rosetta expression cells (Novagen, catalog number: 70954-3CN) were transformed with the prepared pET28b-BE1 or pET28b-BE3 delta UGI and cultured overnight in Luria-Bertani (LB) broth containing 100 µg/ml kanamycin and 50 mg/ml carbenicilin at 37°C. Ten ml of the overnight cultures of Rosetta cells containing pET28b-BE1 or pET28b-BE3 delta UGI was inoculated into 400 ml LB broth containing 100 µg/ml kanamycin and 50 mg/ml carbenicilin and cultured at 30°C until the OD600 reached 0.5-0.6. The cells were cooled to 16°C for 1 hour, supplemented with 0.5 mM IPTG (Isopropyl β-D-1-thiogalactopyranoside), and cultured for 14-18 hours.

For protein purification, cells were harvested by centrifugation at 5,000 x g for 10 min at 4°C and lysed by sonication in 5 ml lysis buffer (50 mM NaH₂PO₄, 300 mM NaCl, 1 mM DTT, and 10 mM imidazole, pH 8.0) supplemented with lysozyme (Sigma) and a protease inhibitor (Roche complete, EDTA-free). The soluble lysate obtained after centrifugation of the cell lysis mixture at 13,000 rpm. for 30 min at 4°C was incubated with Ni-NTA agarose resin (Qiagen) for 1 hour at 4°C. The cell lysate/Ni-NTA mixture was applied to a column and washed with a buffer (50 mM NaH₂PO₄, 300 mM NaCl, and 20 mM imidazole, pH 8.0). The BE3 protein was eluted with an elution buffer (50 mM NaH₂PO₄, 300 mM NaCl, and 250 mM imidazole, pH 8.0). The eluted protein was buffer exchanged with a storage buffer (20 mM HEPES-KOH (pH 7.5), 150 mM KCl, 1 mM DTT, and 20% glycerol) and concentrated with centrifugal filter units (Millipore) to give purified rAPOBEC1-nCas9 protein.

### 3. Deamination of Genomic DNA

Genomic DNA was purified (extracted) from HEK293T cells with a DNeasy Blood & Tissue Kit (Qiagen) according to the manufacturer's instructions. Genomic DNA (10 µg) was incubated with the rAPOBEC1-nCas9 protein (300 nM) purified in Reference Example 2 and an sgRNA (900 nM) in a reaction volume of 500 µL for 8 hours at 37 °C in a buffer (100 mM NaCl, 40 mM Tris-HCl, 10 mM MgCl₂, and 100 µg/ml BSA, pH 7.9).

The used sgRNA was constructed by converting T to U on the overall sequence at an on-target site (on-target sequence; EMX1 on-target sequence; GAGTCCGAGCAGAAGAAGAAGGG (SEQ ID NO: 14)), except the 5'-terminal PAM sequence ((5'-NGG-3') wherein N is A, T, G, or C), and employing the converted sequence as the targeting sequence '(N_{cas9})ₗ' of the following General Formula 3:
5'-(N_{cas9})ₗ-(GUUUUAGAGCUA)-(GAAA)-(UAGCAAGUUAAAAUAAGGCUAGUCCGUUAUCAACUUGAAAAAGUGGCAC CGAGUCGGUGC)-3' (General Formula 3; oligonucleotide linker: GAAA).

After removal of sgRNA using RNase A (50 µg/mL), uracil-containing genomic DNA was purified with a DNeasy Blood & Tissue Kit (Qiagen). The on-target site was amplified by PCR using a SUN-PCR blend and subjected to Sanger sequencing to check BE3-mediated cytosine deamination and USER-mediated DNA cleavage.

### 4. Whole Genome and Digenome Sequencing

Genomic DNA (1 µg) was fragmented to the 400- to 500-bp range using the Covaris system (Life Technologies) and blunt-ended using End Repair Mix (Thermo Fischer). Fragmented DNA was ligated with adapters to produce libraries, which were then subjected to WGS (whole genome sequencing) using HiSeq X Ten Sequencer (Illumina) at Macrogen. (Kim, D., Kim, S., Kim, S., Park, J. & Kim, J.S. Genome-wide target specificities of CRISPR-Cas9 nucleases revealed by multiplex Digenome-seq. Genome research 26, 406-415 (2016)).

### 5. Targeted deep sequencing

On-target and potential off-target sites were amplified with a KAPA HiFi HotStart PCR kit (KAPA Biosystems #KK2501) for deep sequencing library generation. Pooled PCR amplicons were sequenced using MiniSeq (Illumina) or Illumina Miseq (LAS Inc. Korea) with TruSeq HT Dual Index system (Illumina).

Primers used in the targeted deep sequencing are as follows:
EMX1
   On-target sequence: GAGTCCGAGCAGAAGAAGAAGGG (SEQ ID NO: 14) 1^{st} PCR
Forward (5'→3'):
   AGTGTTGAGGCCCCAGTG (SEQ ID NO: 15);
Reverse (5'→3'): 
GTGACTGGAGTTCAGACGTGTGCTCTTCCGATCTCAGCAGCAAGCAG
CACTCT (SEQ ID NO: 16);
 2^{nd} PCR
Forward (5'→3'): 
ACACTCTTTCCCTACACGACGCTCTTCCGATCTGGGCCTCCTGAGTTT
CTCAT (SEQ ID NO: 17);
Reverse (5'→3')

GTGACTGGAGTTCAGACGTGTGCTCTTCCGATCTCAGCAGCAAGCAG
CACTCT (SEQ ID NO: 18).

### EXAMPLE 1: Identification of BE3 off-target site using Digenome-seq

A human genomic DNA was *in vitro* treated with the ribonucleic acid protein in which EMX1-specific sgRNA (see Reference Example 3; on-target sequence: SEQ ID NO: 14) is complexed with rAPOBEC1-nCas9 protein (BE3: purified in Reference Example 2), to induce C→U conversion on one strand and nick formation on the other strand at on-target and off-target sites, followed by performing Digenome-seq with reference to Reference Example 4. In this Example, neither Uracil DNA glycosylase (UDG) nor DNA glycosylase-lyase Endonuclease VIII were used. After end repair and adaptor ligation, the BE3-treated genomic DNA was subjected to whole genome sequencing (WGS).

The procedure is schematically depicted as follows.

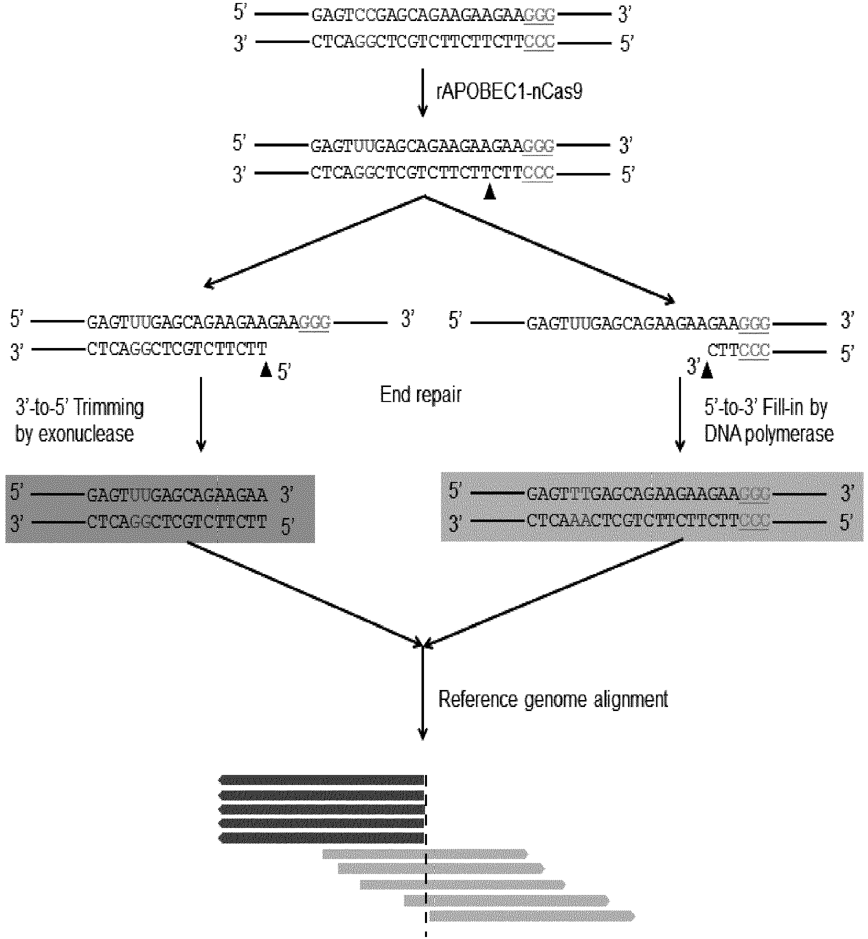

Uniform alignment of sequence reads in one strand and on-target sites of C→U conversion in the other strand, and off-target sites were computationally identified.

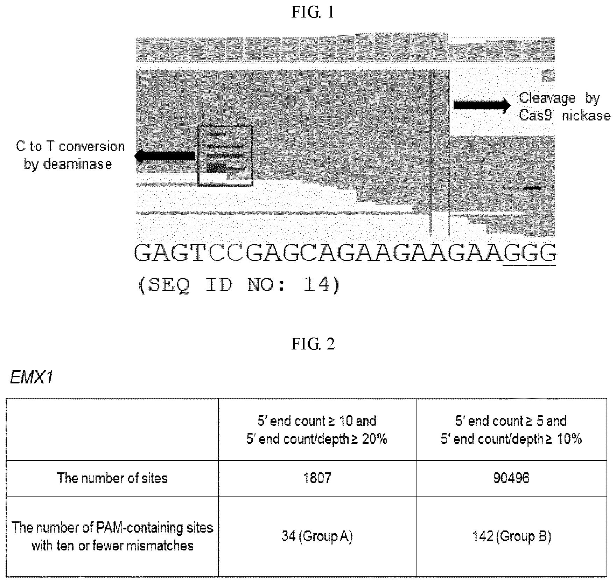

FIG. 1 is a representative IGV image showing straight alignments of sequence reads at the EMX1 on-target site.

In FIG. 2, the number of nicked sites at which sequence reads have uniform alignment only one strand obtained as a result of the Digenome-seq (sites (reads) at which the 5' ends have straight alignment) and the number of PAM-containing sites with 10 or fewer mismatches among the sites are given. Groups A and B were identified to have absolute numbers (n ≥ 10 and 5, respectively) and relative numbers (20% and 10%, respectively)

The absolute number (n≥ 5 or 10) and relative number (10 % or 20 %) of sequence reads with the same 5' ends were counted over the entire human genome to enumerate all sites relevant to uniform alignment patterns in genome. In result, as shown in FIG. 2, 90,496 and 1,807 corresponding sites were acquired. Of the single-strand nicked sites, 34 (Group A) and 142 (Group B; inclusive of Group A) each have a 3-bp PAM (5'-NGN-3' or 5'-NNG-3') downstream of the single-strand nicked sites and show homology to the EMX1 target sequence to an extent of 10 or fewer mismatches.

The Cas9-induced indel frequency and BE3-induced substitution frequency at BE3 off-target sites for EMX1, identified by Digenome-seq, were measured using targeted deep sequencing (see Reference Example 5) in HEK293T cells. From WGS data obtained using intact genomic DNA and rAPOBEC1-nCas9-treated genomic DNA, C→T conversion at each of their sites were investigated.

DNA sequences cleaved by BE3 in EMX1 (1 on-target site + 141 off-target sites = 142 sites in total) are summarized in Table 1, below (in Table 1, on-target sequences and mismatched bases are expressed in lower cases).

**[Table 1]**

| | Chr | Position | DNA seq at a nickase sites | SEQ ID NO: |
|---|---|---|---|---|
| EMX1-001 (on- target) | chr2 | 73160998 | GAGTCCGAGCAGAAGAAGAAGGG | 14 |
| EMX1-002 | chr4 | 131662222 | GAaTCCaAG-AGAAGAAGAATGG | 19 |
| EMX1-003 | chr2 | 219845072 | GAGgCCGAGCAGAAGAAagACGG | 20 |
| EMX1-004 | chr11 | 62365273 | GAaTCCaAGCAGAAGAAGAgAAG | 21 |
| EMX1-005 | chr8 | 128801258 | GAGTCCtAGCAGgAGAAGAAGAG | 22 |
| EMX1-006 | chr15 | 44109763 | GAGTCtaAGCAGAAGAAGAAGAG | 23 |
| EMX1-007 | chrl9 | 24250503 | GAGTCCaAGCAGtAGAgGAAGGG | 24 |
| EMX1-008 | chr6 | 9118799 | acGTCtGAGCAGAAGAAGAATGG | 25 |
| EMX1-009 | chr5 | 9227162 | aAGTCtGAGCAcAAGAAGAATGG | 26 |
| EMX1-010 | chr1 | 4515013 | GtGTCCtAG-AGAAGAAGAAGGG | 27 |
| EMX1-011 | chr5 | 45359067 | GAGTtaGAGCAGAAGAAGAAAGG | 28 |
| EMX1-012 | chr13 | 96928092 | GAGaCaGAG-AGAAGAAGAATGG | 29 |
| EMX1-013 | chr18 | 34906762 | GAGcCtGAGCgGAAGAgGAAAGG | 30 |
| EMX1-014 | chr1 | 184236243 | aAtaCaGAGCAGAAGAAGAATGG | 31 |
| EMX1-015 | chr18 | 1677040 | agtcCaGAGCAaAAtAAGAAGGG | 32 |
| EMX1-016 | chr1 | 33606480 | GAGcCtGAGCAGAAGgAGAAGGG | 33 |
| EMX1-017 | chr3 | 111296327 | GAagaaGAGCAaAAGAAGAAGGG | 34 |
| EMX1-018 | chr22 | 34716275 | GtGaCaGAGCAaAAGAAGAAAGG | 35 |
| EMX1-019 | chr3 | 37781974 | GAagagGAGCAaAAGAAGAAGGG | 36 |
| EMX1-020 | chr20 | 6653999 | aAGTCCagaCAGAAGAAGAAGGA | 37 |
| EMX1-021 | chr16 | 78848850 | aAaTCCaAcCAGAAGAAGAAAGG | 38 |
| EMX1-022 | chr6 | 92449690 | GttcaaGAGCAGgAGAAGAAGGG | 39 |
| EMX1-023 | chr4 | 87256692 | GAGTaaGAGaAGAAGAAGAAGGG | 40 |
| EMX1-024 | chr11 | 43747948 | aAGcCCGAGCAaAgGAAGAAAGG | 41 |
| EMX1-025 | chr5 | 160643032 | cctataGAGCAaAAGAAGAAAGG | 42 |
| EMX1-026 | chr11 | 120873098 | GAtcaaGAGaAGAAGAAGAAGGG | 43 |
| EMX1-027 | chr5 | 62692054 | cAaaaaGAGCAaAAGAAGAACGG | 44 |
| EMX1-028 | chrX | 3077291 | tAcagtGAGCAaAAGAAGAAGGG | 45 |
| EMX1-029 | chr14 | 98236084 | GttcaaGAGCAGgAGAAGAAGGG | 46 |
| EMX1-030 | chr2 | 205473563 | ttcTCaGAGCAaAAGAAGAATGG | 47 |
| EMX1-031 | chr3 | 189633259 | cttTGCcAGGAGAAGgAcAtTGC | 48 |
| EMX1-032 | chr10 | 58498683 | agGTtaGAGCAaAAGAAGAAAGG | 49 |
| EMX1-033 | chr1 | 35818892 | tAtaCgGAGCAGAAGAAGAATGG | 50 |
| EMX1-034 | chr3 | 45605387 | GAGTCCacaCAGAAGAAGAAAGA | 51 |
| EMX1-035 | chr3 | 5031614 | GAaTCCaAGCAGgAGAAGAAGGA | 52 |
| EMX1-036 | chr12 | 106646090 | aAGTCCatGCAGAAGAgGAAGGG | 53 |
| EMX1-037 | chr1 | 23720618 | aAGTCCGAGgAGAgGAAGAAAGG | 54 |
| EMX1-038 | chr11 | 107812992 | aAGTCCaAGt-GAAGAAGAAAGG | 55 |
| EMX1-039 | chr4 | 169444372 | GAGaaCGAGaAGAAagAGgAGAG | 56 |
| EMX1-040 | chr6 | 18327737 | GAGagaGAGagagAGAgGgAGGG | 57 |
| EMX1-041 | chr2 | 230161576 | ctGgCaGAGCAaAAGAAGAgGGG | 58 |
| EMX1-042 | chr3 | 95690186 | tcaTCCaAGCAGAAGAAGAAGAG | 59 |
| EMX1-043 | chr4 | 33321466 | GtacagGAGCAGgAGAAGAATGG | 60 |
| EMX1-044 | chr22 | 49900715 | aAGaagGAGaAGAAGAAGAAGGG | 61 |
| EMX1-045 | chr12 | 94591214 | GAGagaGAGagagAGAgaAAGGG | 62 |
| EMX1-046 | chr5 | 146833190 | GAGcCgGAGCAGAAGAAGgAGGG | 63 |
| EMX1-047 | chr6 | 111509461 | GAGggaGAGagGgAGAgagAAAG | 64 |
| EMX1-048 | chr1 | 26490139 | ttaTCtccGagaAgGAAGAAGGG | 65 |
| EMX1-049 | chr6 | 31265461 | GAtTCtGtcCcGAAtcAGAAGGG | 66 |
| EMX1-050 | chr14 | 30099303 | atGcaaGAGaAGAAGAAGAAAGG | 67 |
| EMX1-051 | chr3 | 83057859 | agcaggGAGCAGAgGAAGAATGG | 68 |
| EMX1-052 | chr15 | 35575311 | GAGaagGAGaAGAAGAAGAAGGG | 69 |
| EMX1-053 | chr1 | 55846672 | actctaGAGCAGAAaAAGAATGG | 70 |
| EMX1-054 | chr6 | 104384459 | GAGgagGAGgAGgAGgAaggAGG | 71 |
| EMX1-055 | chr19 | 9975831 | aAagagGAGaAGAAGAAGAAGGG | 72 |
| EMX1-056 | chr12 | 99525769 | GgGgagGAGCAGAAGAAGAgAGG | 73 |
| EMX1-057 | chr6 | 162280006 | agGcCgagGCAGgAGAAtAgGAG | 74 |
| EMX1-058 | chr7 | 85359110 | GAGaagGAGCAGAAaAAGAATGG | 75 |
| EMX1-059 | chr2 | 10462867 | acagtaGAGCAGAAGAAGAcTGG | 76 |
| EMX1-060 | chr3 | 18195303 | atccaaGAGCAGgAGAAGAAGGG | 77 |
| EMX1-061 | chr2 | 57855994 | ataagaGAGCAaAAGAAGAAAGG | 78 |
| EMX1-062 | chr6 | 33957284 | GAGagaGAGagagAGAgaAACGG | 79 |
| EMX1-063 | chr22 | 37474903 | GAGaagGAGaAGAAGgAGAAGAG | 80 |
| EMX1-064 | chr8 | 141193983 | aAGaagaAGaAGAAGAAGAAGAG | 81 |
| EMX1-065 | chr1 | 110038435 | tttcggGAGCAGAAGAAGAACAG | 82 |
| EMX1-066 | chr4 | 117483357 | atcaCaGAGCAGgAGAAGAAGGG | 83 |
| EMX1-067 | chr4 | 6150362 | aAacagGAGCAGAgGAAGAAGGG | 84 |
| EMX1-068 | chr2 | 116142148 | aAGaagagGaAGAgGAgGAAAAG | 85 |
| EMX1-069 | chr12 | 30794309 | GAaatgGAGaAGAAGAAGAAGGG | 86 |
| EMX1-070 | chr22 | 44527016 | GAGagaGAaagaAAGAAaAAGGA | 87 |
| EMX1-071 | chr9 | 96189722 | GctgtgGAGCAaAAGAAGAAAGG | 88 |
| EMX1-072 | chr8 | 113493465 | GAGgagGAGCAGAAGAAGAAAAG | 89 |
| EMX1-073 | chr11 | 46171476 | tAaaagGAGCAGAAaAAGAAGGG | 90 |
| EMX1-074 | chrX | 3075272 | tAccttGAGCAaAAGAAGAAGGG | 91 |
| EMX1-075 | chr5 | 56038567 | aAGaagGAGaAGAAGAAGAAGGG | 92 |
| EMX1-076 | chr2 | 71789100 | GcaggaGAGCAGAAGAAGAAAGG | 93 |
| EMX1-077 | chr7 | 52389195 | aAGagCGAGattAAGAgGAATGG | 94 |
| EMX1-078 | chr5 | 31088930 | aAGaaaGgagAGgAGAgGAgAGG | 95 |
| EMX1-079 | chr11 | 111680806 | agtagtGAGCAGAAGAAGAtAGG | 96 |
| EMX1-080 | chr20 | 51306677 | aAGaagGAGaAGAAGAAGAAGAG | 97 |
| EMX1-081 | chr19 | 38433655 | GAGagaGAGagagAGAgaAAGAG | 98 |
| EMX1-082 | chr8 | 60956107 | GgccagGAGCAGgAGAAGAAGGG | 99 |
| EMX1-083 | chr16 | 26617803 | agaggaGAGCAGAAGAAGgATGG | 100 |
| EMX1-084 | chr12 | 52621931 | aAGaagGAGaAGAAGAAGgAGGA | 101 |
| EMX1-085 | chr3 | 156028864 | cAtTaaGAGCAGgAGAAGAAGGG | 102 |
| EMX1-086 | chr6 | 40280504 | cgcTgatAcagaAAGAAGAATGG | 103 |
| EMX1-087 | chr1 | 35385601 | GAagtgGAGCAGgAGAAGAAGGG | 104 |
| EMX1-088 | chr1 | 59299359 | tttgtgGAGCAGAAaAAGAAAGG | 105 |
| EMX1-089 | chr15 | 61646877 | aAGTCaGAGgAGAAGAAGAAGGG | 106 |
| EMX1-090 | chr2 | 159685754 | aAagCtGAGCAGAAaAAGAAGGG | 107 |
| EMX1-091 | chr12 | 41494108 | GcagtgGAGCAGAAGAAGAtGGG | 108 |
| EMX1-092 | chr7 | 119831026 | acaaaaGAGCAGAgGAAGAAAGG | 109 |
| EMX1-093 | chr1 | 234492864 | GAagtaGAGCAGAAGAAGAAGCG | 110 |
| EMX1-094 | chr14 | 104091588 | aAagagGgagAGAAGAAGAAGGG | 111 |
| EMX1-095 | chr1 | 31954326 | aAGaagGAGaAGAAGAAGAAGAG | 112 |
| EMX1-096 | chr8 | 120587501 | aAGgCCaAGCAGAAGAgtAATGG | 113 |
| EMX1-097 | chr2 | 46020469 | acacaaGAGCAGAAGAAGAAAGA | 114 |
| EMX1-098 | chr2 | 219294645 | GccaatGAGCAGgAGAAGAAGGG | 115 |
| EMX1-099 | chr8 | 11924153 | cAtataGAGCAaAAGAAGAgAGG | 116 |
| EMX1-100 | chr6 | 54740531 | GAGgtgGAGggGAAGAgGgAAGG | 117 |
| EMX1-101 | chr1 | 156786840 | GAGagaGAGagagAGAgaAAGGG | 118 |
| EMX1-102 | chr6 | 30791217 | aAGgagGAGaAGAAGAAGAAGGG | 119 |
| EMX1-103 | chr3 | 192777993 | GAGggaGAGagagAGAgagAAAG | 120 |
| EMX1-104 | chr2 | 36207879 | agtcggGAGCAGgAGAAGAAAGG | 121 |
| EMX1-105 | chr16 | 54831367 | GttcaaGAGCAGAAGAAGAATGG | 122 |
| EMX1-106 | chr6 | 160868147 | tctaaaGAGCAGAAaAAGAAAGG | 123 |
| EMX1-107 | chr2 | 24438043 | actgatGAGCAGAAGAAGAAAGG | 124 |
| EMX1-108 | chr22 | 37102243 | aAGaagGAGaAGAAGAAGgAGGA | 125 |
| EMX1-109 | chr11 | 121786535 | agGaaaagagAGAAGAAGAAGGG | 126 |
| EMX1-110 | chr7 | 3337380 | GAGgagGAGaAGAAGAAGAAGGG | 127 |
| EMX1-111 | chr8 | 112924257 | GAGagaGAGagagAGAgaAAGGG | 128 |
| EMX1-112 | chr16 | 69047289 | GAGgCCGAagctgAGgtGggAGG | 129 |
| EMX1-113 | chr8 | 105164125 | GAGcCCaAGaAGAAGAAGAAGGA | 130 |
| EMX1-114 | chr13 | 83353702 | atGTaCagagAGAAGAAGAAAGG | 131 |
| EMX1-115 | chr2 | 102929260 | GccTtCagagAGAAGAAGAATGG | 132 |
| EMX1-116 | chr15 | 22366621 | GgagtaGAGCAGAgGAAGAAGGG | 133 |
| EMX1-117 | chr2 | 172374203 | GAagtaGAGCAGAAGAAGAAGCG | 134 |
| EMX1-118 | chr8 | 31096390 | GctcCtGAGCAGAAGAAGAACAG | 135 |
| EMX1-119 | chr2 | 66729772 | agtTCaGAGCAGgAGAAGAATGG | 136 |
| EMX1-120 | chr2 | 14472327 | atGaaCagagAGAAGAAGAATGG | 137 |
| EMX1-121 | chr8 | 140468447 | GAGagCGAGagagAGAgagAGGG | 138 |
| EMX1-122 | chr7 | 52204863 | aAaaagGAGCAGAAGAAGAAGGA | 139 |
| EMX1-123 | chr1 | 151027598 | ttcTCCaAGCAGAAGAAGAAGAG | 140 |
| EMX1-124 | chr1 | 35590719 | GAGagaGAGagagAGAgaAAGGG | 141 |
| EMX1-125 | chr1 | 106744880 | ttGgaaagagAGAAGAAGAAGGG | 142 |
| EMX1-126 | chr10 | 115484209 | aAGaggaAGaAGAAGAAGAAGAG | 143 |
| EMX1-127 | chr3 | 119686684 | GAGagaGAGaAagAGAAagAGAG | 144 |
| EMX1-128 | chr8 | 53295601 | GAagaaGAGaAGAAGAAGAAGGG | 145 |
| EMX1-129 | chr18 | 12032247 | GAtTCtGAGaAaAttAAGAtGGG | 146 |
| EMX1-130 | chr15 | 61383748 | GgGctCcgGCAGAAGAtGccATG | 147 |
| EMX1-131 | chr1 | 209298672 | GAtTCCaAGCAatgGAgGAgGGG | 148 |
| EMX1-132 | chr7 | 17446438 | GtccaaGAGCAGgAGAAGAAGGG | 149 |
| EMX1-133 | chr13 | 74473871 | atcTggGAGCAGgAGAAGAAGGG | 150 |
| EMX1-134 | chr5 | 5141237 | GAGgatccGagGAtGtAGAAGGG | 151 |
| EMX1-135 | chr12 | 5041728 | GAagaaGAagAaAgaAAGAAAGA | 152 |
| EMX1-136 | chr8 | 112756160 | cAGagaGAGaAtAAGtAGcATAG | 153 |
| EMX1-137 | chr8 | 17384135 | tgaggaagagAGAAGAAGAAAGG | 154 |
| EMX1-138 | chr12 | 4545932 | cAagCatgagAGAAGAAGAtGGG | 155 |
| EMX1-139 | chr10 | 58848728 | GAGcaCGAGCAagAGAAGAAGGG | 156 |
| EMX1-140 | chr14 | 48932119 | GAGTCCcAGCAaAAGAAGAAAAG | 157 |
| EMX1-141 | chr3 | 145057362 | GAGTCCct-CAGgAGAAGAAAGG | 158 |
| EMX1-142 | chr9 | 111348573 | GAGTCCttG-AGAAGAAGgAAGG | 159 |

Counts (numbers of sequence reads having the same 5' end), depths (numbers of sequence reads at specific sites), % (count/depth), and counts of reads with C→T conversion, which were all measured at the nicked sites enumerated in Table 1, are summarized in Table 2, below:

**[Table 2]**

| | count | depth | %(count/ depth) | C to T conversion | | Group A | Group B |
|---|---|---|---|---|---|---|---|
| | | | | (+) Base editor | Untreated | | |
| EMX1-001 (on- target) | 21 | 51 | 41.2 | 6 | 0 | v | v |
| EMX1-002 | 21 | 39 | 53.8 | 8 | 0 | v | v |
| EMX1-003 | 22 | 41 | 53.7 | 0 | 0 | v | v |
| EMX1-004 | 36 | 79 | 45.6 | 10 | 0 | v | v |
| EMX1-005 | 29 | 68 | 42.6 | 1 | 0 | v | v |
| EMX1-006 | 26 | 62 | 41.9 | 9 | 0 | v | v |
| EMX1-007 | 10 | 29 | 34.5 | 0 | 0 | v | v |
| EMX1-008 | 24 | 86 | 27.9 | 0 | 0 | v | v |
| EMX1-009 | 44 | 159 | 27.7 | 10 | 0 | v | v |
| EMX1-010 | 11 | 41 | 26.8 | 0 | 0 | v | v |
| EMX1-011 | 50 | 109 | 45.9 | N.A. | N.A. | v | v |
| EMX1-012 | 15 | 43 | 34.9 | 1 | 0 | v | v |
| EMX1-013 | 16 | 46 | 34.8 | 0 | 0 | v | v |
| EMX1-014 | 22 | 64 | 34.4 | 0 | 1 | v | v |
| EMX1-015 | 16 | 53 | 30.2 | 0 | 0 | v | v |
| EMX1-016 | 19 | 63 | 30.2 | 1 | 0 | v | v |
| EMX1-017 | 24 | 82 | 29.3 | N.A. | N.A. | v | v |
| EMX1-018 | 24 | 85 | 28.2 | 0 | 0 | v | v |
| EMX1-019 | 14 | 50 | 28.0 | N.A. | N.A. | v | v |
| EMX1-020 | 10 | 36 | 27.8 | 0 | 0 | v | v |
| EMX1-021 | 13 | 47 | 27.7 | 0 | 0 | v | v |
| EMX1-022 | 13 | 48 | 27.1 | 1 | 0 | v | v |
| EMX1-023 | 10 | 37 | 27.0 | N.A. | N.A. | v | v |
| EMX1-024 | 11 | 42 | 26.2 | 0 | 0 | v | v |
| EMX1-025 | 15 | 58 | 25.9 | N.A. | N.A. | v | v |
| EMX1-026 | 11 | 43 | 25.6 | 0 | 0 | v | v |
| EMX1-027 | 16 | 67 | 23.9 | N.A. | N.A. | v | v |
| EMX1-028 | 10 | 44 | 22.7 | N.A. | N.A. | v | v |
| EMX1-029 | 10 | 45 | 22.2 | 0 | 0 | v | v |
| EMX1-030 | 14 | 63 | 22.2 | 0 | 0 | v | v |
| EMX1-031 | 13 | 61 | 21.3 | 0 | 0 | v | v |
| EMX1-032 | 13 | 61 | 21.3 | N.A. | N.A. | v | v |
| EMX1-033 | 14 | 66 | 21.2 | 0 | 0 | v | v |
| EMX1-034 | 14 | 53 | 26.4 | 2 | 0 | v | v |
| EMX1-035 | 9 | 48 | 18.8 | 0 | 0 | - | v |
| EMX1-036 | 8 | 46 | 17.4 | 1 | 0 | - | v |
| EMX1-037 | 8 | 51 | 15.7 | 0 | 0 | - | v |
| EMX1-038 | 6 | 42 | 14.3 | 0 | 0 | - | v |
| EMX1-039 | 7 | 22 | 31.8 | 1 | 0 | - | v |
| EMX1-040 | 7 | 22 | 31.8 | N.A. | N.A. | - | v |
| EMX1-041 | 7 | 23 | 30.4 | 0 | 0 | - | v |
| EMX1-042 | 7 | 25 | 28.0 | 0 | 0 | - | v |
| EMX1-043 | 6 | 23 | 26.1 | 0 | 0 | - | v |
| EMX1-044 | 7 | 27 | 25.9 | N.A. | N.A. | - | v |
| EMX1-045 | 8 | 35 | 22.9 | N.A. | N.A. | - | v |
| EMX1-046 | 9 | 40 | 22.5 | 0 | 0 | - | v |
| EMX1-047 | 8 | 38 | 21.1 | N.A. | N.A. | - | v |
| EMX1-048 | 5 | 24 | 20.8 | 0 | 0 | - | v |
| EMX1-049 | 7 | 34 | 20.6 | 0 | 0 | - | v |
| EMX1-050 | 8 | 40 | 20.0 | 0 | 0 | - | v |
| EMX1-051 | 6 | 30 | 20.0 | N.A. | N.A. | - | v |
| EMX1-052 | 10 | 51 | 19.6 | N.A. | N.A. | - | v |
| EMX1-053 | 12 | 63 | 19.0 | 0 | 0 | - | v |
| EMX1-054 | 7 | 37 | 18.9 | N.A. | N.A. | - | v |
| EMX1-055 | 12 | 64 | 18.8 | N.A. | N.A. | - | v |
| EMX1-056 | 8 | 43 | 18.6 | N.A. | N.A. | - | v |
| EMX1-057 | 5 | 27 | 18.5 | 1 | 0 | - | v |
| EMX1-058 | 9 | 49 | 18.4 | N.A. | N.A. | - | v |
| EMX1-059 | 13 | 71 | 18.3 | N.A. | N.A. | - | v |
| EMX1-060 | 10 | 55 | 18.2 | 0 | 0 | - | v |
| EMX1-061 | 10 | 55 | 18.2 | N.A. | N.A. | - | v |
| EMX1-062 | 5 | 28 | 17.9 | N.A. | N.A. | - | v |
| EMX1-063 | 5 | 28 | 17.9 | N.A. | N.A. | - | v |
| EMX1-064 | 7 | 40 | 17.5 | N.A. | N.A. | - | v |
| EMX1-065 | 13 | 76 | 17.1 | 0 | 0 | - | v |
| EMX1-066 | 5 | 30 | 16.7 | 0 | 0 | - | v |
| EMX1-067 | 5 | 30 | 16.7 | 0 | 0 | - | v |
| EMX1-068 | 6 | 36 | 16.7 | N.A. | N.A. | - | v |
| EMX1-069 | 19 | 115 | 16.5 | N.A. | N.A. | - | v |
| EMX1-070 | 6 | 37 | 16.2 | N.A. | N.A. | - | v |
| EMX1-071 | 9 | 56 | 16.1 | N.A. | N.A. | - | v |
| EMX1-072 | 15 | 94 | 16.0 | N.A. | N.A. | - | v |
| EMX1-073 | 11 | 70 | 15.7 | N.A. | N.A. | - | v |
| EMX1-074 | 7 | 45 | 15.6 | 0 | 0 | - | v |
| EMX1-075 | 9 | 59 | 15.3 | N.A. | N.A. | - | v |
| EMX1-076 | 9 | 59 | 15.3 | N.A. | N.A. | - | v |
| EMX1-077 | 5 | 33 | 15.2 | 0 | 0 | - | v |
| EMX1-078 | 14 | 93 | 15.1 | N.A. | N.A. | - | v |
| EMX1-079 | 6 | 40 | 15.0 | N.A. | N.A. | - | v |
| EMX1-080 | 11 | 75 | 14.7 | N.A. | N.A. | - | v |
| EMX1-081 | 6 | 42 | 14.3 | N.A. | N.A. | - | v |
| EMX1-082 | 6 | 43 | 14.0 | 0 | 0 | - | v |
| EMX1-083 | 6 | 43 | 14.0 | N.A. | N.A. | - | v |
| EMX1-084 | 7 | 50 | 14.0 | N.A. | N.A. | - | v |
| EMX1-085 | 7 | 50 | 14.0 | N.A. | N.A. | - | v |
| EMX1-086 | 5 | 36 | 13.9 | N.A. | N.A. | - | v |
| EMX1-087 | 7 | 51 | 13.7 | N.A. | N.A. | - | v |
| EMX1-088 | 7 | 51 | 13.7 | N.A. | N.A. | - | v |
| EMX1-089 | 6 | 44 | 13.6 | 0 | 0 | - | v |
| EMX1-090 | 10 | 74 | 13.5 | 0 | 0 | - | v |
| EMX1-091 | 12 | 89 | 13.5 | N.A. | N.A. | - | v |
| EMX1-092 | 5 | 37 | 13.5 | N.A. | N.A. | - | v |
| EMX1-093 | 7 | 52 | 13.5 | N.A. | N.A. | - | v |
| EMX1-094 | 6 | 45 | 13.3 | N.A. | N.A. | - | v |
| EMX1-095 | 6 | 46 | 13.0 | N.A. | N.A. | - | v |
| EMX1-096 | 11 | 85 | 12.9 | 0 | 0 | - | v |
| EMX1-097 | 6 | 47 | 12.8 | 0 | 0 | - | v |
| EMX1-098 | 5 | 39 | 12.8 | N.A. | N.A. | - | v |
| EMX1-099 | 6 | 48 | 12.5 | N.A. | N.A. | - | v |
| EMX1-100 | 6 | 48 | 12.5 | N.A. | N.A. | - | v |
| EMX1-101 | 8 | 64 | 12.5 | N.A. | N.A. | - | v |
| EMX1-102 | 7 | 57 | 12.3 | N.A. | N.A. | - | v |
| EMX1-103 | 6 | 50 | 12.0 | N.A. | N.A. | - | v |
| EMX1-104 | 7 | 59 | 11.9 | 0 | 0 | - | v |
| EMX1-105 | 6 | 51 | 11.8 | 0 | 0 | - | v |
| EMX1-106 | 9 | 77 | 11.7 | N.A. | N.A. | - | v |
| EMX1-107 | 8 | 69 | 11.6 | N.A. | N.A. | - | v |
| EMX1-108 | 5 | 43 | 11.6 | N.A. | N.A. | - | v |
| EMX1-109 | 5 | 43 | 11.6 | N.A. | N.A. | - | v |
| EMX1-110 | 7 | 61 | 11.5 | N.A. | N.A. | - | v |
| EMX1-111 | 7 | 61 | 11.5 | N.A. | N.A. | - | v |
| EMX1-112 | 5 | 44 | 11.4 | 0 | 0 | - | v |
| EMX1-113 | 5 | 44 | 11.4 | 0 | 0 | - | v |
| EMX1-114 | 7 | 62 | 11.3 | 0 | 0 | - | v |
| EMX1-115 | 6 | 53 | 11.3 | 0 | 0 | - | v |
| EMX1-116 | 8 | 71 | 11.3 | N.A. | N.A. | - | v |
| EMX1-117 | 6 | 53 | 11.3 | N.A. | N.A. | - | v |
| EMX1-118 | 6 | 54 | 11.1 | 0 | 0 | - | v |
| EMX1-119 | 5 | 45 | 11.1 | 0 | 0 | - | v |
| EMX1-120 | 5 | 46 | 10.9 | 0 | 0 | - | v |
| EMX1-121 | 6 | 55 | 10.9 | 0 | 0 | - | v |
| EMX1-122 | 6 | 55 | 10.9 | N.A. | N.A. | - | v |
| EMX1-123 | 8 | 75 | 10.7 | 0 | 0 | - | v |
| EMX1-124 | 6 | 56 | 10.7 | N.A. | N.A. | - | v |
| EMX1-125 | 7 | 66 | 10.6 | N.A. | N.A. | - | v |
| EMX1-126 | 5 | 47 | 10.6 | N.A. | N.A. | - | v |
| EMX1-127 | 5 | 47 | 10.6 | N.A. | N.A. | - | v |
| EMX1-128 | 8 | 76 | 10.5 | N.A. | N.A. | - | v |
| EMX1-129 | 5 | 48 | 10.4 | 0 | 0 | - | v |
| EMX1-130 | 5 | 48 | 10.4 | 0 | 0 | - | v |
| EMX1-131 | 5 | 48 | 10.4 | 0 | 0 | - | v |
| EMX1-132 | 5 | 48 | 10.4 | 1 | 0 | - | v |
| EMX1-133 | 5 | 48 | 10.4 | N.A. | N.A. | - | v |
| EMX1-134 | 7 | 68 | 10.3 | 0 | 0 | - | v |
| EMX1-135 | 6 | 59 | 10.2 | N.A. | N.A. | - | v |
| EMX1-136 | 5 | 49 | 10.2 | N.A. | N.A. | - | v |
| EMX1-137 | 7 | 69 | 10.1 | N.A. | N.A. | - | v |
| EMX1-138 | 5 | 50 | 10.0 | 0 | 0 | - | v |
| EMX1-139 | 5 | 50 | 10.0 | 0 | 0 | - | v |
| EMX1-140 | 7 | 44 | 15.9 | 0 | 0 | - | v |
| EMX1-141 | 5 | 40 | 12.5 | 1 | 0 | - | v |
| EMX1-142 | 6 | 49 | 12.2 | 1 | 0 | - | v |

| | | | | | | | |
|---|---|---|---|---|---|---|---|
| (N.A.: not applicable because there are no cytosines to be deaminated at these sites) | | | | | | | |

As can be seen in Table 2, the WGS data obtained using the BE-3-treated genomic DNA and intact (BE-3 untreated) genomic DNA showed the observation of C→T conversion at 16 sites (BE-3 treated) and 1 site (BE-3 untreated) among 142 sites of Group B. Of these sites, 70 sites do not contain cytosine at positions 4 to 8, which is a window of BE3-mediated deamination (numbered 1 to 20 in the 5' to 3' direction (expressed as N. A. in Table 2).

In order to assess the off-target effect in parts of Groups A and B sites identified Digenome-seq, DNA from HEK293T cells was subjected to targeted deep sequencing and measured for BE3-induced base editing frequencies and Cas9-induced indel frequencies. The results are given in Table 3, below:

**[Table 3]**

| | Validation by NGS | | | | | |
|---|---|---|---|---|---|---|
| | Indel frequency (%) | | | Base editing frequency (%) | | |
| | (-) Cas9 | (+) Cas9 | Validation | (-) BE3 | (+) BE3 | Validation |
| EMX1-001 (on- target) | 0.15 | 61.59 | Validated | 0.10 | 49.33 | Validated |
| EMX1-002 | 0.01 | 0.01 | Invalidated | 0.16 | 1.05 | Validated |
| EMX1-003 | 0.00 | 7.94 | Validated | 0.24 | 4.04 | Validated |
| EMX1-004 | 0.00 | 0.01 | Validated | 0.16 | 0.93 | Validated |
| EMX1-005 | 0.00 | 8.63 | Validated | 0.05 | 2.47 | Validated |
| EMX1-006 | 0.29 | 38.25 | Validated | 0.04 | 15.59 | Validated |
| EMX1-007 | 0.01 | 0.01 | Invalidated | 0.08 | 0.13 | Validated |
| EMX1-008 | 0.02 | 0.17 | Validated | 0.03 | 0.62 | Validated |
| EMX1-009 | 0.10 | 3.45 | Validated | 0.02 | 0.15 | Validated |
| EMX1-010 | 0.08 | 0.08 | Invalidated | 0.07 | 0.70 | Validated |
| EMX1-034 | 0.00 | 0.00 | Invalidated | 0.33 | 0.40 | Invalidated |
| EMX1-035 | 0.46 | 0.89 | Validated | 0.23 | 0.48 | Validated |
| EMX1-036 | 0.01 | 0.02 | Invalidated | 0.09 | 0.31 | Validated |
| EMX1-037 | 0.01 | 0.23 | Validated | 0.20 | 0.23 | Validated |
| EMX1-038 | 0.01 | 0.01 | Invalidated | 0.14 | 0.16 | Validated |
| EMX1-140 | 0.01 | 0.00 | Invalidated | 0.38 | 0.36 | Invalidated |
| EMX1-141 | 0.00 | 0.00 | Invalidated | 0.30 | 0.37 | Invalidated |
| EMX1-142 | 0.01 | 0.01 | Invalidated | 0.19 | 0.17 | Invalidated |

As is understood from data of Table 3, a total of 18 sites were analyzed and BE3-induced point mutations were observed at 14 sites including the EMX1 on-target sites, with frequencies above noise levels caused by sequencing errors (0.002∼0.38%) (at a validation rate of 78%). It is possible that BE3 can induce mutagenesis at the other BE3-associated, Digenome-captured sites with frequencies below background noise levels. Notably, the method is able to identify BE3 off-target sites at which base editing was detected with a frequency of 0.13% or less, demonstrating that Digenome-seq is a highly sensitive method. EMX1-specific Cas9 nucleases induced indels at 9 of 18 sites with frequencies above noise levels, indicating that BE3 and Cas9 off-target sites are often different from each other. Taken together, such results suggest that BE3 off-target sites can be identified using the Digenome-seq data.

As described above, it will be understood by a person having ordinary skill in the technical field to which the present disclosure pertains that the present disclosure may be embodied in other specific forms without departing from the technical spirit or essential characteristics thereof. In this regard, it should be understood that the above-described embodiments are intended to illustrate in every aspect, but are not intended to be limiting. The scope of the invention should be construed to cover all modifications and variations that come within the meaning and range, as well as equivalent concepts thereof, as defined by the appended claims rather than the foregoing description.

## Claims

1. A method for identifying an off-target site of a cytidine deaminase, the method comprising:
(i) introducing into a cell or contacting with DNA separated from a cell a cytidine deaminase or a gene encoding a cytidine deaminase, an inactivated target-specific endonuclease or a gene encoding an inactivated target-specific endonuclease, and a guide RNA or a gene encoding a guide RNA, to induce a single-strand break in the DNA; and
(ii) analyzing a nucleic acid sequence of the single-strand cleaved DNA fragment; and
(iii) identifying a single-strand break site from the nucleic acid sequence data obtained by the analysis.
wherein the inactivated target-specific endonuclease is a Cas9 protein or Cpfl protein lacking endonuclease activity of cleaving a DNA duplex.

2. A method for analyzing a nucleic acid sequence of DNA to which base editing is introduced by a cytidine deaminase, the method comprising the steps of:
(i) introducing into a cell or contacting with DNA separated from a cell a cytidine deaminase or a gene encoding a cytidine deaminase, an inactivated target-specific endonuclease or a gene encoding an inactivated target-specific endonuclease, and a guide RNA or a gene encoding a guide RNA, to induce a single-strand break in the DNA; and
(ii) analyzing a nucleic acid sequence of a DNA fragment in which the single-strand break has been induced,
wherein the inactivated target-specific endonuclease is a Cas9 protein or Cpfl protein lacking the endonuclease activity of cleaving a DNA duplex.

3. A method for identifying a base-editing site of a cytidine deaminase, the method comprising the steps of:
(i) introducing to a cell or contacting with DNA separated from a cell a cytidine deaminase or a gene encoding a cytidine deaminase, an inactivated target-specific endonuclease or a gene encoding an inactivated target-specific endonuclease, and a guide RNA or a gene encoding a guide RNA, to induce a single-strand break in DNA;
(ii) analyzing a nucleic acid sequence of the DNA fragment in which the single-strand break has been induced; and
(iii) identifying a single-strand break site from the sequence reads obtained by the analysis,
wherein the inactivated target-specific endonuclease is a Cas9 protein or Cpfl protein lacking the endonuclease activity of cleaving a DNA duplex.

4. The method of any one of claims 1 to 3, wherein the inactivated target-specific endonuclease is a *Streptococcus pyogenes-derived* Cas9 protein which is different in amino acid residue from the wild-type protein at the following positions:
(1) D10, H840, or D10 and H840;
(2) at least one selected from the group consisting of D1135, R1335, and T1337; or
(3) both of the positions of (1) and (2).

5. The method of any one of claims 1 to 4, wherein the guide RNA is a doublestranded or single-stranded guide RNA (sgRNA) including CRISPR RNA (crRNA) and *trans*-activating crRNA (tracrRNA).

6. The method of any one of claims 1 to 5, wherein, the cytidine deaminase is APOBEC (apolipoprotein B mRNA editing enzyme, catalytic polypeptide-like), AID (activation-induced cytidine deaminase), CDA (cytidine deaminase), or a combination thereof.

7. The method of any one of claims 1 to 6, wherein the cytidine deaminase and the inactivated target-specific endonuclease are used
in a fusion protein form,
in a mixture of the cytidine deaminase or an mRNA coding therefor and the inactivated target-specific endonuclease or an mRNA coding therefore, or
in a form of a plasmid carrying both a cytidine deaminase-encoding gene and an inactivated target-specific endonuclease-encoding gene or plasmids carrying a cytidine deaminase-encoding gene and an inactivated target-specific endonuclease-encoding gene, respectively.

8. The method of any one of claims 1 to 7, wherein the method does not use a uracil-specific excision reagent (USER), which is a uracil DNA glycosylase (DG), endonuclease VIII, or a combination thereof.

9. The method of any one of claims 1 to 8, being carried out *in vitro.*

10. The method of any one of claims 1 to 9, wherein the DNA separated from cells are a genomic DNA.

11. The method of any one of claims 1 to 10, wherein the DNA separated from cells in step (i) is a genomic DNA and the nucleic acid sequence analysis of step (iii) is carried out by whole genome sequencing.

12. The method of claims 1, further comprising a step of determining the cleaved site as an off-target site when the cleaved site is not an on-target site, after step (iv).

13. The method of claim 12, wherein the step (iii) includes a step of identifying a number of DNA reads of which the 5' ends are vertically aligned.

14. The method of claim 13, wherein the off-target site has two or more DNA reads of which the 5' ends are vertically aligned.

15. The method of claim 14, wherein the off-target site corresponds to at least one of the following conditions:
a strand complementary to the strand on which a break has been induced in a DNA fragment includes a PAM sequence;
a complementary strand to the strand on which a break has been induced in a DNA fragment includes 15 or fewer nucleotide mismatches with a sequence at the on-target site; and
a complementary strand to the strand on which a break has been induced in a DNA fragment includes conversion of at least cytosine (C) residue to uracil (U) or thymine (T).


<110>	INSTITUTE FOR BASIC SCIENCE

<120>	METHOD OF IDENTIFYING GENOME-WIDE OFF-TARGET SITES OF BASE
	EDITORS BY DETECTING SINGLE STRAND BREAKS IN GENOMIC DNA

<130>	OPP20175578KR

<150>	62/446,951
<151>	2017-01-17

<160>	18

<170>	KopatentIn 3.0

<210>	1
<211>	12
<212>	RNA
<213>	Artificial Sequence

<220>
<223>	Essential part of crRNA


<400>	1
guuuuagagc ua	12


<210>	2
<211>	10
<212>	RNA
<213>	Artificial Sequence

<220>
<223>	3'-terminal part of crRNA


<400>	2
ugcuguuuug	10


<210>	3
<211>	60
<212>	RNA
<213>	Artificial Sequence

<220>
<223>	Essential part of tracrRNA


<400>	3

uagcaaguua aaauaaggcu aguccguuau caacuugaaa aaguggcacc gagucggugc 60

<210> 4
<211> 1368
<212> PRT
<213> Artificial Sequence

<220>
<223> Cas9 from Streptococcus pyogenes

<400> 4

<210>    5
<211>    4107
<212>    DNA
<213>    Artificial Sequence

<220>
<223>    Cas9-coding sequence

<400>    5
atggacaaga agtacagcat cggcctggac atcggtacca acagcgtggg ctgggccgtg        60

atcaccgacg agtacaaggt gcccagcaag aagttcaagg tgctgggcaa caccgaccgc       120

cacagcatca agaagaacct gatcggcgcc ctgctgttcg acagcggcga gaccgccgag       180

gccacccgcc tgaagcgcac cgcccgccgc cgctacaccc gccgcaagaa ccgcatctgc       240

tacctgcagg agatcttcag caacgagatg gccaaggtgg acgacagctt cttccaccgc       300

ctggaggaga gcttcctggt ggaggaggac aagaagcacg agcgccaccc catcttcggc       360

aacatcgtgg acgaggtggc ctaccacgag aagtacccca ccatctacca cctgcgcaag       420

aagctggtgg acagcaccga caaggccgac ctgcgcctga tctacctggc cctggcccac       480

atgatcaagt tccgcggcca cttcctgatc gagggcgacc tgaaccccga caacagcgac       540

gtggacaagc tgttcatcca gctggtgcag acctacaacc agctgttcga ggagaacccc 600

atcaacgcca gcggcgtgga cgccaaggcc atcctgagcg cccgcctgag caagagccgc 660

cgcctggaga acctgatcgc ccagctgccc ggcgagaaga agaacggcct gttcggcaac 720

ctgatcgccc tgagcctggg cctgaccccc aacttcaaga gcaacttcga cctggccgag 780

gacgccaagc tgcagctgag caaggacacc tacgacgacg acctggacaa cctgctggcc 840

cagatcggcg accagtacgc cgacctgttc ctggccgcca agaacctgag cgacgccatc 900

ctgctgagcg acatcctgcg cgtgaacacc gagatcacca aggcccccct gagcgccagc 960

atgatcaagc gctacgacga gcaccaccag gacctgaccc tgctgaaggc cctggtgcgc 1020

cagcagctgc ccgagaagta caaggagatc ttcttcgacc agagcaagaa cggctacgcc 1080

ggctacatcg acggcggcgc cagccaggag gagttctaca agttcatcaa gcccatcctg 1140

gagaagatgg acggcaccga ggagctgctg gtgaagctga accgcgagga cctgctgcgc 1200

aagcagcgca ccttcgacaa cggcagcatc ccccaccaga tccacctggg cgagctgcac 1260

gccatcctgc gccgccagga ggacttctac cccttcctga aggacaaccg cgagaagatc 1320

gagaagatcc tgaccttccg catcccctac tacgtgggcc ccctggcccg cggcaacagc 1380

cgcttcgcct ggatgacccg caagagcgag gagaccatca ccccctggaa cttcgaggag 1440

gtggtggaca agggcgccag cgcccagagc ttcatcgagc gcatgaccaa cttcgacaag 1500

aacctgccca acgagaaggt gctgcccaag cacagcctgc tgtacgagta cttcaccgtg 1560

tacaacgagc tgaccaaggt gaagtacgtg accgagggca tgcgcaagcc cgccttcctg 1620

agcggcgagc agaagaaggc catcgtggac ctgctgttca agaccaaccg caaggtgacc 1680

gtgaagcagc tgaaggagga ctacttcaag aagatcgagt gcttcgacag cgtggagatc 1740

agcggcgtgg aggaccgctt caacgccagc ctgggcacct accacgacct gctgaagatc 1800

atcaaggaca aggacttcct ggacaacgag gagaacgagg acatcctgga ggacatcgtg 1860

ctgaccctga ccctgttcga ggaccgcgag atgatcgagg agcgcctgaa gacctacgcc 1920

cacctgttcg acgacaaggt gatgaagcag ctgaagcgcc gccgctacac cggctggggc 1980

cgcctgagcc gcaagcttat caacggcatc cgcgacaagc agagcggcaa gaccatcctg 2040

gacttcctga agagcgacgg cttcgccaac cgcaacttca tgcagctgat ccacgacgac 2100

agcctgacct tcaaggagga catccagaag gcccaggtga gcggccaggg cgacagcctg 2160

cacgagcaca tcgccaacct ggccggcagc cccgccatca agaagggcat cctgcagacc 2220

gtgaaggtgg tggacgagct ggtgaaggtg atgggccgcc acaagcccga gaacatcgtg 2280

atcgagatgg cccgcgagaa ccagaccacc cagaagggcc agaagaacag ccgcgagcgc 2340

atgaagcgca tcgaggaggg catcaaggag ctgggcagcc agatcctgaa ggagcacccc 2400

gtggagaaca cccagctgca gaacgagaag ctgtacctgt actacctgca gaacggccgc 2460

gacatgtacg tggaccagga gctggacatc aaccgcctga gcgactacga cgtggaccac 2520

atcgtgcccc agagcttcct gaaggacgac agcatcgaca acaaggtgct gacccgcagc 2580

gacaagaacc gcggcaagag cgacaacgtg cccagcgagg aggtggtgaa gaagatgaag 2640

aactactggc gccagctgct gaacgccaag ctgatcaccc agcgcaagtt cgacaacctg 2700

accaaggccg agcgcggcgg cctgagcgag ctggacaagg ccggcttcat caagcgccag 2760

ctggtggaga cccgccagat caccaagcac gtggcccaga tcctggacag ccgcatgaac 2820

accaagtacg acgagaacga caagctgatc cgcgaggtga aggtgatcac cctgaagagc 2880

aagctggtga gcgacttccg caaggacttc cagttctaca aggtgcgcga gatcaacaac 2940

taccaccacg cccacgacgc ctacctgaac gccgtggtgg gcaccgccct gatcaagaag 3000

taccccaagc tggagagcga gttcgtgtac ggcgactaca aggtgtacga cgtgcgcaag 3060

atgatcgcca agagcgagca ggagatcggc aaggccaccg ccaagtactt cttctacagc 3120

aacatcatga acttcttcaa gaccgagatc accctggcca acggcgagat ccgcaagcgc 3180

cccctgatcg agaccaacgg cgagaccggc gagatcgtgt gggacaaggg ccgcgacttc 3240

gccaccgtgc gcaaggtgct gagcatgccc caggtgaaca tcgtgaagaa gaccgaggtg 3300

cagaccggcg gcttcagcaa ggagagcatc ctgcccaagc gcaacagcga caagctgatc 3360

gcccgcaaga aggactggga ccccaagaag tacggcggct tcgacagccc caccgtggcc 3420

tacagcgtgc tggtggtggc caaggtggag aagggcaaga gcaagaagct gaagagcgtg 3480

aaggagctgc tgggcatcac catcatggag cgcagcagct tcgagaagaa ccccatcgac 3540

ttcctggagg ccaagggcta caaggaggtg aagaaggacc tgatcatcaa gctgcccaag 3600

tacagcctgt tcgagctgga gaacggccgc aagcgcatgc tggccagcgc cggcgagctg 3660

cagaagggca acgagctggc cctgcccagc aagtacgtga acttcctgta cctggccagc 3720

cactacgaga agctgaaggg cagccccgag gacaacgagc agaagcagct gttcgtggag 3780

cagcacaagc actacctgga cgagatcatc gagcagatca gcgagttcag caagcgcgtg 3840

atcctggccg acgccaacct ggacaaggtg ctgagcgcct acaacaagca ccgcgacaag 3900

cccatccgcg agcaggccga gaacatcatc cacctgttca ccctgaccaa cctgggcgcc 3960

cccgccgcct tcaagtactt cgacaccacc atcgaccgca agcgctacac cagcaccaag 4020

gaggtgctgg acgccaccct gatccaccag agcatcaccg gtctgtacga gacccgcatc 4080

gacctgagcc agctgggcgg cgactaa 4107

<210> 6
<211> 229
<212> PRT
<213> Artificial Sequence

<220>
<223> APOBEC-1 from Rattus norvegicus

<400> 6

<210>   7
<211>   5148
<212>   DNA
<213>   Artificial Sequence

<220>
<223>   BE3 coding sequence

<400>   7
catcatcatc atcatcacat gtcttctgaa accggtccgg ttgcggttga cccgaccctg      60

cgtcgtcgta tcgaaccgca cgaattcgaa gttttcttcg acccgcgtga actgcgtaaa     120

gaaacctgcc tgctgtacga aatcaactgg ggtggtcgtc actctatctg gcgtcacacc     180

tctcagaaca ccaacaaaca cgttgaagtt aacttcatcg aaaaattcac caccgaacgt     240

tacttctgcc cgaacacccg ttgctctatc acctggttcc tgtcttggtc tccgtgcggt     300

gaatgctctc gtgcgatcac cgaattcctg tctcgttacc cgcacgttac cctgttcatc     360

tacatcgcgc gtctgtacca ccacgcggac ccgcgtaacc gtcagggtct gcgtgacctg     420

atctcttctg gtgttaccat ccagatcatg accgaacagg aatctggtta ctgctggcgt 480

aacttcgtta actactctcc gtctaacgaa gcgcactggc cgcgttaccc gcacctgtgg 540

gttcgtctgt acgttctgga actgtactgc atcatcctgg gtctgccgcc gtgcctgaac 600

atcctgcgtc gtaaacagcc gcagctgacc ttcttcacca tcgcgctgca gtcttgccac 660

taccagcgtc tgccgccgca catcctgtgg gcgaccggtc tgaaatccgg tagcgaaaca 720

ccggggactt cagaatcggc caccccggag tctgataaga aatactcaat aggcttagct 780

atcggcacaa atagcgtcgg atgggcggtg atcactgatg aatataaggt tccgtctaaa 840

aagttcaagg ttctgggaaa tacagaccgc cacagtatca aaaaaaatct tataggggct 900

cttttatttg acagtggaga gacagcggaa gcgactcgtc tcaaacggac agctcgtaga 960

aggtatacac gtcggaagaa tcgtatttgt tatctacagg agattttttc aaatgagatg 1020

gcgaaagtag atgatagttt ctttcatcga cttgaagagt cttttttggt ggaagaagac 1080

aagaagcatg aacgtcatcc tatttttgga aatatagtag atgaagttgc ttatcatgag 1140

aaatatccaa ctatctatca tctgcgaaaa aaattggtag attctactga taaagcggat 1200

ttgcgcttaa tctatttggc cttagcgcat atgattaagt ttcgtggtca ttttttgatt 1260

gagggagatt taaatcctga taatagtgat gtggacaaac tatttatcca gttggtacaa 1320

acctacaatc aattatttga agaaaaccct attaacgcaa gtggagtaga tgctaaagcg 1380

attctttctg cacgattgag taaatcaaga cgattagaaa atctcattgc tcagctcccc 1440

ggtgagaaga aaaatggctt atttgggaat ctcattgctt tgtcattggg tttgacccct 1500

aattttaaat caaattttga tttggcagaa gatgctaaat tacagctttc aaaagatact 1560

tacgatgatg atttagataa tttattggcg caaattggag atcaatatgc tgatttgttt 1620

ttggcagcta agaatttatc agatgctatt ttactttcag atatcctaag agtaaatact 1680

gaaataacta aggctcccct atcagcttca atgattaaac gctacgatga acatcatcaa 1740

gacttgactc ttttaaaagc tttagttcga caacaacttc cagaaaagta taaagaaatc 1800

ttttttgatc aatcaaaaaa cggatatgca ggttatattg atgggggagc tagccaagaa 1860

gaattttata aatttatcaa accaatttta gaaaaaatgg atggtactga ggaattattg 1920

gtgaaactaa atcgtgaaga tttgctgcgc aagcaacgga cctttgacaa cggctctatt 1980

ccccatcaaa ttcacttggg tgagctgcat gctattttga gaagacaaga agacttttat 2040

ccatttttaa aagacaatcg tgagaagatt gaaaaaatct tgacttttcg aattccttat 2100

tatgttggtc cattggcgcg tggcaatagt cgttttgcat ggatgactcg gaagtctgaa 2160

gaaacaatta ccccatggaa ttttgaagaa gttgtcgata aaggtgcttc agctcaatca 2220

tttattgaac gcatgacaaa ctttgataaa aatcttccaa atgaaaaagt actaccaaaa 2280

catagtttgc tttatgagta ttttacggtt tataacgaat tgacaaaggt caaatatgtt 2340

actgaaggaa tgcgaaaacc agcatttctt tcaggtgaac agaagaaagc cattgttgat 2400

ttactcttca aaacaaatcg aaaagtaacc gttaagcaat taaaagaaga ttatttcaaa 2460

aaaatagaat gttttgatag tgttgaaatt tcaggagttg aagatagatt taatgcttca 2520

ttaggtacct accatgattt gctaaaaatt attaaagata aagatttttt ggataatgaa 2580

gaaaatgaag atatcttaga ggatattgtt ttaacattga ccttatttga agatagggag 2640

atgattgagg aaagacttaa aacatatgct cacctctttg atgataaggt gatgaaacag 2700

cttaaacgtc gccgttatac tggttgggga cgtttgtctc gaaaattgat taatggtatt 2760

agggataagc aatctggcaa aacaatatta gatttttgga aatcagatgg ttttgccaat 2820

cgcaatttta tgcagctgat ccatgatgat agtttgacat ttaaagaaga cattcaaaaa 2880

gcacaagtgt ctggacaagg cgatagttta catgaacata ttgcaaattt agctggtagc 2940

cctgctatta aaaaaggtat tttacagact gtaaaagttg ttgatgaatt ggtcaaagta 3000

atggggcggc ataagccaga aaatatcgtt attgaaatgg cacgtgaaaa tcagacaact 3060

caaaagggcc agaaaaattc gcgagagcgt atgaaacgaa tcgaagaagg tatcaaagaa 3120

ttaggaagtc agattcttaa agagcatcct gttgaaaata ctcaattgca aaatgaaaag 3180

ctctatctct attatctcca aaatggaaga gacatgtatg tggaccaaga attagatatt 3240

aatcgtttaa gtgattatga tgtcgatcac attgttccac aaagtttcct taaagacgat 3300

tcaatagaca ataaggtctt aacgcgttct gataaaaatc gtggtaaatc ggataacgtt 3360

ccaagtgaag aagtagtcaa aaagatgaaa aactattgga gacaacttct aaacgccaag 3420

ttaatcactc aacgtaagtt tgataattta acgaaagctg aacgtggagg tttgagtgaa 3480

cttgataaag ctggttttat caaacgccaa ttggttgaaa ctcgccaaat cactaagcat 3540

gtggcacaaa ttttggatag tcgcatgaat actaaatacg atgaaaatga taaacttatt 3600

cgagaggtta aagtgattac cttaaaatct aaattagttt ctgacttccg aaaagatttc 3660

caattctata aagtacgtga gattaacaat taccatcatg cccatgatgc gtatctaaat 3720

gccgtcgttg gaactgcttt gattaagaaa tatccaaaac ttgaatcgga gtttgtctat 3780

ggtgattata aagtttatga tgttcgtaaa atgattgcta agtctgagca agaaataggc 3840

aaagcaaccg caaaatattt cttttactct aatatcatga acttcttcaa aacagaaatt 3900

acacttgcaa atggagagat tcgcaaacgc cctctaatcg aaactaatgg ggaaactgga 3960

gaaattgtct gggataaagg gcgagatttt gccacagtgc gcaaagtatt gtccatgccc 4020

caagtcaata ttgtcaagaa aacagaagta cagacaggcg gattctccaa ggagtcaatt 4080

ttaccaaaaa gaaattcgga caagcttatt gctcgtaaaa aagactggga tccaaaaaaa 4140

tatggtggtt ttgatagtcc aacggtagct tattcagtcc tagtggttgc taaggtggaa 4200

aaagggaaat cgaagaagtt aaaatccgtt aaagagttac tagggatcac aattatggaa 4260

agaagttcct ttgaaaaaaa tccgattgac tttttagaag ctaaaggata taaggaagtt 4320

aaaaaagact taatcattaa actacctaaa tatagtcttt ttgagttaga aaacggtcgt 4380

aaacggatgc tggctagtgc cggagaatta caaaaaggaa atgagctggc tctgccaagc 4440

aaatatgtga atttttatat tttagctagt cattatgaaa agttgaaggg tagtccagaa 4500

gataacgaac aaaaacaatt gtttgtggag cagcataagc attatttaga tgagattatt 4560

gagcaaatca gtgaattttc taagcgtgtt attttagcag atgccaattt agataaagtt 4620

cttagtgcat ataacaaaca tagagacaaa ccaatacgtg aacaagcaga aaatattatt 4680

catttattta cgttgacgaa tcttggagct ccagccgcat tcaagtattt tgacacaacg 4740

atagatcgca aacgatacac ttctaccaag gaggtgctag acgcgacact gattcaccaa 4800

tccatcacgg gattatatga aactcggata gatttgtcac agcttggggg tgactctggt 4860

ggttctacta atctgtcaga tattattgaa aaggagaccg gtaagcaact ggttatccag 4920

gaatccatcc tcatgctccc agaggaggtg gaagaagtca ttgggaacaa gccggaaagc 4980

gatatactcg tgcacaccgc ctacgacgag agcaccgacg agaatgtcat gcttctgact 5040

agcgacgccc ctgaatacaa gccttgggct ctggtcatac aggatagcaa cggtgagaac 5100

aagattaaga tgctctctgg tggttctccc aagaagaaga ggaaagtc 5148

<210> 8
<211> 18
<212> DNA
<213> Artificial Sequence

<220>
<223> Hisx6

<400> 8
catcatcatc atcatcac 18

<210> 9
<211> 687
<212> DNA
<213> Artificial Sequence

<220>
<223> rAPOBEC1

<400> 9
atgtcttctg aaaccggtcc ggttgcggtt gacccgaccc tgcgtcgtcg tatcgaaccg 60

cacgaattcg aagttttctt cgacccgcgt gaactgcgta aagaaacctg cctgctgtac 120

gaaatcaact ggggtggtcg tcactctatc tggcgtcaca cctctcagaa caccaacaaa 180

cacgttgaag ttaacttcat cgaaaaattc accaccgaac gttacttctg cccgaacacc 240

cgttgctcta tcacctggtt cctgtcttgg tctccgtgcg gtgaatgctc tcgtgcgatc 300

accgaattcc tgtctcgtta cccgcacgtt accctgttca tctacatcgc gcgtctgtac 360

caccacgcgg acccgcgtaa ccgtcagggt ctgcgtgacc tgatctcttc tggtgttacc 420

atccagatca tgaccgaaca ggaatctggt tactgctggc gtaacttcgt taactactct 480

ccgtctaacg aagcgcactg gccgcgttac ccgcacctgt gggttcgtct gtacgttctg 540

gaactgtact gcatcatcct gggtctgccg ccgtgcctga acatcctgcg tcgtaaacag 600

ccgcagctga ccttcttcac catcgcgctg cagtcttgcc actaccagcg tctgccgccg 660

cacatcctgt gggcgaccgg tctgaaa 687

<210> 10
<211> 48
<212> DNA
<213> Artificial Sequence

<220>
<223> XTEN (linker)

<400> 10
tccggtagcg aaacaccggg gacttcagaa tcggccaccc cggagtct 48

<210> 11
<211> 4101
<212> DNA
<213> Artificial Sequence

<220>
<223> coding gene of Cas9 (D10A, nickase)

<400> 11
gataagaaat actcaatagg cttagctatc ggcacaaata gcgtcggatg ggcggtgatc 60

actgatgaat ataaggttcc gtctaaaaag ttcaaggttc tgggaaatac agaccgccac 120

agtatcaaaa aaaatcttat aggggctctt ttatttgaca gtggagagac agcggaagcg 180

actcgtctca aacggacagc tcgtagaagg tatacacgtc ggaagaatcg tatttgttat 240

ctacaggaga ttttttcaaa tgagatggcg aaagtagatg atagtttctt tcatcgactt 300

gaagagtctt ttttggtgga agaagacaag aagcatgaac gtcatcctat ttttggaaat 360

atagtagatg aagttgctta tcatgagaaa tatccaacta tctatcatct gcgaaaaaaa 420

ttggtagatt ctactgataa agcggatttg cgcttaatct atttggcctt agcgcatatg 480

attaagtttc gtggtcattt tttgattgag ggagatttaa atcctgataa tagtgatgtg 540

gacaaactat ttatccagtt ggtacaaacc tacaatcaat tatttgaaga aaaccctatt 600

aacgcaagtg gagtagatgc taaagcgatt ctttctgcac gattgagtaa atcaagacga 660

ttagaaaatc tcattgctca gctccccggt gagaagaaaa atggcttatt tgggaatctc 720

attgctttgt cattgggttt gacccctaat tttaaatcaa attttgattt ggcagaagat 780

gctaaattac agctttcaaa agatacttac gatgatgatt tagataattt attggcgcaa 840

attggagatc aatatgctga tttgtttttg gcagctaaga atttatcaga tgctatttta 900

ctttcagata tcctaagagt aaatactgaa ataactaagg ctcccctatc agcttcaatg 960

attaaacgct acgatgaaca tcatcaagac ttgactcttt taaaagcttt agttcgacaa 1020

caacttccag aaaagtataa agaaatcttt tttgatcaat caaaaaacgg atatgcaggt 1080

tatattgatg ggggagctag ccaagaagaa ttttataaat ttatcaaacc aattttagaa 1140

aaaatggatg gtactgagga attattggtg aaactaaatc gtgaagattt gctgcgcaag 1200

caacggacct ttgacaacgg ctctattccc catcaaattc acttgggtga gctgcatgct 1260

attttgagaa gacaagaaga cttttatcca tttttaaaag acaatcgtga gaagattgaa 1320

aaaatcttga cttttcgaat tccttattat gttggtccat tggcgcgtgg caatagtcgt 1380

tttgcatgga tgactcggaa gtctgaagaa acaattaccc catggaattt tgaagaagtt 1440

gtcgataaag gtgcttcagc tcaatcattt attgaacgca tgacaaactt tgataaaaat 1500

cttccaaatg aaaaagtact accaaaacat agtttgcttt atgagtattt tacggtttat 1560

aacgaattga caaaggtcaa atatgttact gaaggaatgc gaaaaccagc atttctttca 1620

ggtgaacaga agaaagccat tgttgattta ctcttcaaaa caaatcgaaa agtaaccgtt 1680

aagcaattaa aagaagatta tttcaaaaaa atagaatgtt ttgatagtgt tgaaatttca 1740

ggagttgaag atagatttaa tgcttcatta ggtacctacc atgatttgct aaaaattatt 1800

aaagataaag attttttgga taatgaagaa aatgaagata tcttagagga tattgtttta 1860

acattgacct tatttgaaga tagggagatg attgaggaaa gacttaaaac atatgctcac 1920

ctctttgatg ataaggtgat gaaacagctt aaacgtcgcc gttatactgg ttggggacgt 1980

ttgtctcgaa aattgattaa tggtattagg gataagcaat ctggcaaaac aatattagat 2040

tttttgaaat cagatggttt tgccaatcgc aattttatgc agctgatcca tgatgatagt 2100

ttgacattta aagaagacat tcaaaaagca caagtgtctg gacaaggcga tagtttacat 2160

gaacatattg caaatttagc tggtagccct gctattaaaa aaggtatttt acagactgta 2220

aaagttgttg atgaattggt caaagtaatg gggcggcata agccagaaaa tatcgttatt 2280

gaaatggcac gtgaaaatca gacaactcaa aagggccaga aaaattcgcg agagcgtatg 2340

aaacgaatcg aagaaggtat caaagaatta ggaagtcaga ttcttaaaga gcatcctgtt 2400

gaaaatactc aattgcaaaa tgaaaagctc tatctctatt atctccaaaa tggaagagac 2460

atgtatgtgg accaagaatt agatattaat cgtttaagtg attatgatgt cgatcacatt 2520

gttccacaaa gtttccttaa agacgattca atagacaata aggtcttaac gcgttctgat 2580

aaaaatcgtg gtaaatcgga taacgttcca agtgaagaag tagtcaaaaa gatgaaaaac 2640

tattggagac aacttctaaa cgccaagtta atcactcaac gtaagtttga taatttaacg 2700

aaagctgaac gtggaggttt gagtgaactt gataaagctg gttttatcaa acgccaattg 2760

gttgaaactc gccaaatcac taagcatgtg gcacaaattt tggatagtcg catgaatact 2820

aaatacgatg aaaatgataa acttattcga gaggttaaag tgattacctt aaaatctaaa 2880

ttagtttctg acttccgaaa agatttccaa ttctataaag tacgtgagat taacaattac 2940

catcatgccc atgatgcgta tctaaatgcc gtcgttggaa ctgctttgat taagaaatat 3000

ccaaaacttg aatcggagtt tgtctatggt gattataaag tttatgatgt tcgtaaaatg 3060

attgctaagt ctgagcaaga aataggcaaa gcaaccgcaa aatatttctt ttactctaat 3120

atcatgaact tcttcaaaac agaaattaca cttgcaaatg gagagattcg caaacgccct 3180

ctaatcgaaa ctaatgggga aactggagaa attgtctggg ataaagggcg agattttgcc 3240

acagtgcgca aagtattgtc catgccccaa gtcaatattg tcaagaaaac agaagtacag 3300

acaggcggat tctccaagga gtcaatttta ccaaaaagaa attcggacaa gcttattgct 3360

cgtaaaaaag actgggatcc aaaaaaatat ggtggttttg atagtccaac ggtagcttat 3420

tcagtcctag tggttgctaa ggtggaaaaa gggaaatcga agaagttaaa atccgttaaa 3480

gagttactag ggatcacaat tatggaaaga agttcctttg aaaaaaatcc gattgacttt 3540

ttagaagcta aaggatataa ggaagttaaa aaagacttaa tcattaaact acctaaatat 3600

agtctttttg agttagaaaa cggtcgtaaa cggatgctgg ctagtgccgg agaattacaa 3660

aaaggaaatg agctggctct gccaagcaaa tatgtgaatt ttttatattt agctagtcat 3720

tatgaaaagt tgaagggtag tccagaagat aacgaacaaa aacaattgtt tgtggagcag 3780

cataagcatt atttagatga gattattgag caaatcagtg aattttctaa gcgtgttatt 3840

ttagcagatg ccaatttaga taaagttctt agtgcatata acaaacatag agacaaacca 3900

atacgtgaac aagcagaaaa tattattcat ttatttacgt tgacgaatct tggagctcca 3960

gccgcattca agtattttga cacaacgata gatcgcaaac gatacacttc taccaaggag 4020

gtgctagacg cgacactgat tcaccaatcc atcacgggat tatatgaaac tcggatagat 4080

ttgtcacagc ttgggggtga c 4101

<210> 12
<211> 21
<212> DNA
<213> Artificial Sequence

<220>
<223> NLS

<400> 12
cccaagaaga agaggaaagt c 21

<210> 13
<211> 12
<212> DNA
<213> Artificial Sequence

<220>
<223> linker

<400> 13
tctggtggtt ct 12

<210> 14
<211> 23
<212> DNA
<213> Artificial Sequence

<220>
<223>    EMX1 on-target sequence

<400>    14
gagtccgagc agaagaagaa ggg                                                23

<210>    15
<211>    18
<212>    DNA
<213>    Artificial Sequence

<220>
<223>    1st PCR forward primer for targeted deep sequencing

<400>    15
agtgttgagg ccccagtg                                                      18

<210>    16
<211>    53
<212>    DNA
<213>    Artificial Sequence

<220>
<223>    1st PCR reverse primer for targeted deep sequencing

<400>    16
gtgactggag ttcagacgtg tgctcttccg atctcagcag caagcagcac tct               53

<210>    17
<211>    53
<212>    DNA
<213>    Artificial Sequence

<220>
<223>    2nd PCR forward primer for targeted deep sequencing

<400>    17
acactctttc cctacacgac gctcttccga tctgggcctc ctgagtttct cat               53

<210>    18
<211>    53

<212>	DNA
<213>	Artificial Sequence

<220>
<223>	2nd PCR reverse primer for targeted deep sequencing

<400>	18
gtgactggag ttcagacgtg tgctcttccg atctcagcag caagcagcac tct	53